# EUROPÄISCHE PATENTSCHRIFT

(11) **EP 1 613 744 B1**
(45) Veröffentlichungstag und Bekanntmachung des Hinweises auf die Patenterteilung: **09.09.2015**
(21) Anmeldenummer: 04725899.1
(22) Anmeldetag: 06.04.2004
(51) Int. Cl.: C12N 9/02, C12P 7/64, A01H 1/00, C12N 15/00, A01K 67/00, A23D 9/00, A23K 1/16

(54) **DELTA-4-DESATURASEN AUS EUGLENA GRACILIS, EXPRIMIERENDE PFLANZEN UND PUFA ENTHALTENDE ÖLE**
DELTA-4 DESATURASES FROM EUGLENA GRACILIS, EXPRESSING PLANTS, AND OILS CONTAINING PUFA
PLANTES EXPRIMANT DES DELTA-4-DESATURASES PROVENANT D'EUGLENA GRACILIS ET HUILES CONTENANT DES ACIDES GRAS POLYINSATURES (PUFA)

(30) Priorität: 08.04.2003 DE 10316267
(43) Veröffentlichungstag der Anmeldung: 11.01.2006
(73) Patentinhaber: BASF Plant Science GmbH, 67056 Ludwigshafen (DE)
(72) Erfinder: CIRPUS, Petra, 68163 Mannheim (DE); BAUER, Jörg, 67063 Ludwigshafen (DE); MEYER, Astrid, 22767 Hamburg (DE); HEINZ, Ernst, 22609 Hamburg (DE); ZÄHRINGER, Ulrich, 22926 Ahrensburg (DE)
(74) Vertreter: Lutz, Andreas
(86) Internationale Anmeldenummer: PCT/EP2004/003628
(87) Internationale Veröffentlichungsnummer: WO 2004/090123

(56) Entgegenhaltungen:
- WO-A-02/26946
- WO-A-02/090493
- PEREIRA SUZETTE L ET AL: "Recent advances in the study of fatty acid desaturases from animals and lower eukaryotes." PROSTAGLANDINS LEUKOTRIENES AND ESSENTIAL FATTY ACIDS, Bd. 68, Nr. 2, Februar 2003 (2003-02), Seiten 97-106, XP002298342 ISSN: 0952-3278
- BARSANTI LAURA ET AL: "Fatty acid content in wild type and WZSL mutant of Euglena gracilis: Effects of carbon source and growth conditions" JOURNAL OF APPLIED PHYCOLOGY, Bd. 12, Nr. 3-5, Oktober 2000 (2000-10), Seiten 515-520, XP002298343 ISSN: 0921-8971
- LOPEZ ALONSO D ET AL: "Plants as 'chemical factories' for the production of polyunsaturated fatty acids" BIOTECHNOLOGY ADVANCES, ELSEVIER PUBLISHING, BARKING, GB, Bd. 18, Nr. 6, Oktober 2000 (2000-10), Seiten 481-497, XP004218725 ISSN: 0734-9750
- WALLIS J G ET AL: "The DELTA8-desaturase of Euglena gracilis: An alternate pathway for synthesis of 20-carbon polyunsaturated fatty acids" ARCHIVES OF BIOCHEMISTRY AND BIOPHYSICS, NEW YORK, US, US, Bd. 365, Nr. 2, 15. Mai 1999 (1999-05-15), Seiten 307-316, XP002291433 ISSN: 0003-9861
- MEYER ASTRID ET AL: "Biosynthesis of docosahexaenoic acid in Euglena gracilis: Biochemical and molecular evidence for the involvement of a DELTA4-fatty acyl group desaturase." BIOCHEMISTRY, Bd. 42, Nr. 32, 19. August 2003 (2003-08-19), Seiten 9779-9788, XP002298344 ISSN: 0006-2960
- SCHEEDER MARTIN R L ET AL: "Effect of PUFA at sn-2 position in dietary triacylglycerols on the fatty acid composition of adipose tissues in non-ruminant farm animals.", EUROPEAN JOURNAL OF LIPID SCIENCE AND TECHNOLOGY, vol. 105, no. 2, February 2003 (2003-02), pages 74-82, ISSN: 1438-7697

## Beschreibung

Die vorliegende Erfindung betrifft ein verbessertes Verfahren zur spezifischen Herstellung von ungesättigten ω-3 Fettsäuren sowie ein Verfahren zur Herstellung von Triglyceriden mit einem erhöhten Gehalt an ungesättigten Fettsäuren, besonders von ω-3 Fettsäuren mit mehr als drei Doppelbindungen. Die Erfindung betrifft die Herstellung eines transgenen nicht-humanen Organismus bevorzugt einer transgenen Pflanze oder eines transgenen Mikroorganismus mit erhöhtem Gehalt an Fettsäuren, Ölen oder Lipiden mit Δ-4-Doppelbindungen aufgrund der Expression einer Δ-4-Desaturase aus Euglena gracilis.

Außerdem betrifft die Erfindung Expressionskassetten enthaltend eine Nukleinsäuresequenz, einen Vektor und Organismen enthaltend mindestens eine Nukleinsäuresequenz bzw. eine Expressionskassette. Außerdem betrifft die Erfindung ungesättigte Fettsäuren sowie Triglyceride mit einem erhöhten Gehalt an ungesättigten Fettsäuren und deren Verwendung.

Fettsäuren und Triglyceride haben eine Vielzahl von Anwendungen in der Lebensmittelindustrie, der Tierernährung, der Kosmetik und im Pharmabereich. Je nachdem ob es sich um freie gesättigte oder ungesättigte Fettsäuren oder um Triglyceride mit einem erhöhten Gehalt an gesättigten oder ungesättigten Fettsäuren handelt, sind sie für die unterschiedlichsten Anwendungen geeignet.

Mehrfach ungesättigte langkettige ω3-Fettsäuren wie Eicosapentaensäure (EPA) oder Docosahexaensäure (DPA) sind wichtige Komponenten der menschlichen Ernährung aufgrund ihrer verschiedenen Rollen in der Gesundheit, die Aspekte wie die Entwicklung des kindlichen Gehirns, der Funktionalität des Auges, der Synthese von Hormonen und anderer Signalstoffe, sowie die Vorbeugung von Herz-Kreislauf-Beschwerden, Krebs und Diabetes umfassen (Poulos, A Lipids 30:1-14, 1995; Horrocks, LA und Yeo YK Pharmacol Res 40:211-225, 1999). Es besteht aus diesem Grund ein Bedarf an der Produktion mehrfach ungesättigter langkettiger Fettsäuren.

So werden beispielsweise mehrfach ungesättigte Fettsäuren Babynahrung zur Erhöhung des Nährwertes zugesetzt, sowie zur ungehinderten Entwicklung des Säuglings.. Hauptsächlich werden die verschiedenen Fettsäuren und Triglyceride aus Mikroorganismen wie Mortierella oder aus Öl-produzierenden Pflanzen wie Soja, Raps, Sonnenblume und weiteren gewonnen, wobei sie in der Regel in Form ihrer Triacylglyceride anfallen. In höheren Pflanzen treten allerdings keine langkettigen ungesättigten Fettsäuren auf. Die langkettigen Fettsäuren stammen zum größten Teil aus Fischöl bzw. aus der Fermentation von entsprechenden Algen (z.B. Thraustochytrium) bzw. Pilzen (z.B. Mortierella). Die freien Fettsäuren werden vorteilhaft durch Verseifung hergestellt.

Je nach Anwendungszweck sind Öle mit gesättigten oder ungesättigten Fettsäuren bevorzugt, so sind z.B. in der humanen Ernährung Lipide mit ungesättigten Fettsäuren speziell mehrfach ungesättigten Fettsäuren bevorzugt, da sie einen positiven Einfluss auf den Cholesterinspiegel im Blut und damit auf die Möglichkeit einer Herzerkrankung haben. Sie finden in verschiedenen diätischen Lebensmitteln oder Medikamenten Anwendung.

Aufgrund ihrer positiven Eigenschaften hat es in der Vergangenheit nicht an Ansätzen gefehlt, Gene, die an der Synthese von Fettsäuren bzw. Triglyceriden beteiligt sind, für die Herstellung von Ölen in verschiedenen Organismen mit geändertem Gehalt an ungesättigten Fettsäuren verfügbar zu machen. So wird in WO 91/13972 und seinem US-Äquivalent eine Δ-9-Desaturase beschrieben. In WO 93/11245 wird eine delta-15-Desaturase in WO 94/11516 wird eine Δ-12-Desaturase beansprucht. Weitere Desaturasen werden beispielsweise in EP-A-0 550162, WO 94/18337, WO 97/30582, WO 97/21340, WO 95/18222, EP-A-0 794 250, Stukey et al., J. Biol. Chem., 265, 1990: 20144-20149, Wada et al., Nature 347, 1990: 200-203 oder Huang et al., Lipids 34, 1999: 649-659 beschrieben. Die biochemische Charakterisierung der verschiedenen Desaturasen ist jedoch bisher nur unzureichend erfolgt, da die Enzyme als membrangebundene Proteine nur sehr schwer zu isolieren und charakterisieren sind (McKeon et al., Methods in Enzymol. 71, 1981: 12141-12147, Wang et al., Plant Physiol. Biochem., 26,1988: 777-792). In der Regel erfolgt die Charakterisierung membrangebundener Desaturasen durch Einbringung in einen geeigneten Organismus, der anschließend auf Enzymaktivität mittels Edukt- und Produktanalyse untersucht wird. Δ -6-Desaturasen werden in WO 93/06712, US 5,614,393, US5614393, WO 96/21022, WO0021557 und WO 99/27111 beschrieben und auch die Anwendung zur Produktion in transgenen Organismen beschrieben wie in WO9846763 WO9846764, WO9846765. Dabei wird auch die Expression verschiedener Desaturasen wie in WO9964616 oder WO9846776 und Bildung polyungesättigter Fettsäuren beschrieben und beansprucht.

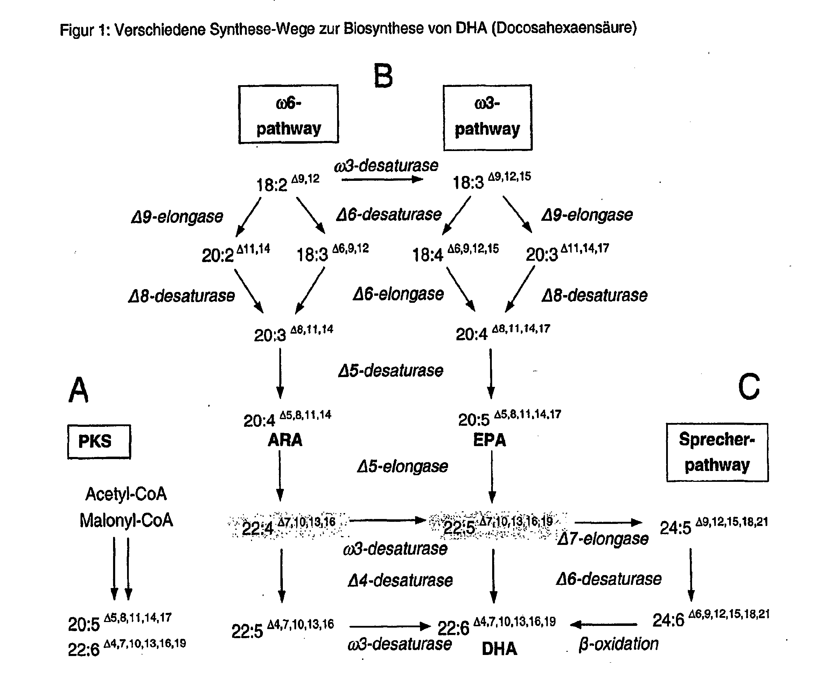

Für die Synthese von Docosahexaensäure (DHA) werden verschiedene Synthesewege diskutiert (Fig. 1). So erfolgt die Produktion von DHA in marinen Bakterien wie Vibrio sp. oder Shewanella sp. nach dem Polyketid-Weg (Yu, R. et al. Lipids 35:1061-1064, 2000; Takeyama, H. et al. Microbiology 143:2725-2731, 1197)).

Ein alternative Strategie verläuft über die wechselnde Aktivität von Desaturasen und Elongasen (Zank, T.K. et al. Plant Journal 31:255-268, 2002; Sakuradani, E. et al. Gene 238:445-453,1999). Der letzte Schritt stellt dabei das Einfügen der Doppelbindung in Position C4-C5 durch eine Δ4-Desaturase dar. In diesem Zusammenhang wurde von Sprecher et al. (Voss, A. et al. Journal of Biological Chemistry 266:19995-20000,1991) demonstriert, dass DHA auch unabhängig einer Δ-4-Desaturase in Rattenlebern synthetisiert werden kann. Für die Herstellung in Pflanzen und Mikroorganismen ist der sogenannte Sprecher-Syntheseweg (siehe Figur 1) allerdings nicht geeignet, da die Regulationsmechanismen, die der α-Oxidation zugrunde liegen, nicht bekannt sind.

Verschiedene Δ4-Desaturasen sind bereits in WO200226946 und WO2002090493 beschrieben.

Bzgl. der Effektivität der Expression von Desaturasen und ihrem Einfluss auf die Bildung polyungesättigter Fettsäuren ist anzumerken, dass durch Expression der entsprechenden Desaturase wie bisher beschrieben (siehe oben) lediglich geringe Gehalte an delta-4 ungesättigten Fettsäuren/Lipiden erreicht wurden. Des weiteren ist für die obengenannten Enzyme keine Spezifität für die ernährungsphysiologisch wichtige sn-2 Position in Glycerolipiden beschrieben (Hunter, JE, Lipids 36(7):655-668, 2001).

Nach wie vor besteht daher ein großer Bedarf an neuen und besser geeigneten Genen, die für Enzyme kodieren, die an der Biosynthese ungesättigter Fettsäuren beteiligt sind und es ermöglichen, bestimmte Fettsäuren spezifisch in einem technischen Maßstab herzustellen, ohne dass unerwünschte Nebenprodukte entstehen. Bei der Auswahl von Biosynthesegenen sind vor allem zwei Merkmale besonders wichtig. Zum einen besteht nach wie vor ein Bedarf an verbesserten Verfahren zur Gewinnung möglichst hoher Gehalte an polyungesättigten Fettsäuren. Zum anderen sollten die eingesetzten Enzyme hoch spezifisch für ein bestimmtes Substrat sein, da möglichst keine ungewünschten Nebenprodukte entstehen dürfen, die möglicherweise negative oder bisher nicht erforschte physiologische Effekte in der Nahrungsmittelanwendung haben.

Um eine Anreicherung der Nahrung und des Futters mit diesen spezifisch hergestellten, mehrfach ungesättigten Fettsäuren zu ermöglichen, besteht daher ein großer Bedarf an einem einfachen, kostengünstigen Verfahren zur Herstellung dieser mehrfach ungesättigten Fettsäuren mit Hilfe von möglichst spezifischen Enzymen, die an der Fettsäurebiosynthese beteiligt sind.

Es bestand daher die Aufgabe für die Herstellung von mehrfach ungesättigten Fettsäuren in einem Organismus vorteilhaft in einem eukaryontischen nicht-humanen Organismus bevorzugt in einer Pflanze neue Nukleinsäuren zu isolieren, die möglichst spezifisch an der Synthese dieser mehrfach ungesättigten Fettsäuren beteiligt sind. Diese Aufgabe wurde durch die erfindungsgemäßen isolierten Nukleinsäuresequenzen gelöst, die für Polypeptide mit Δ-4-Desaturaseaktivität codieren, ausgewählt aus der Gruppe:
a) einer Nukleinsäuresequenz mit der in SEQ ID NO: 1 dargestellten Sequenz,
b) Nukleinsäuresequenzen, die sich als Ergebnis des degenerierten genetischen Codes von der in SEQ ID NO: 1 enthaltenden codierenden Sequenz ableiten lassen, oder
c) Derivate der in SEQ ID NO: 1 dargestellten Nukleinsäuresequenz, die für Polypeptide mit der in SEQ ID NO: 2 dargestellten Aminosäuresequenzencodieren und mindestens 40 % Homologie auf Aminosäureebene mit SEQ ID NO: 2 aufweisen und eine Δ-4-Desaturaseaktivität aufweisen.

Überraschenderweise wurde gefunden, dass eine Δ4-Desaturasen aus Euglena gracilis besonders spezifisch für die Umsetzung von Docopentaensäure (DPA) zu Docohexaensäure (DHA) ist, wenn sie in einem heterologen System exprimiert werden. Damit kann Docosahexaensäure in Pflanzen oder Microorganismen hergestellt werden, wobei die Spezifität des gefundenen Enzyms das Entstehen von unerwünschten Nebenprodukten stark reduziert. Dabei wird die Doppelbindung an Position C4-C5 der Fettsäure nur eingefügt, wenn in Position C7-C8 bereits eine Doppelbindung vorhanden ist. Das gefundene Enzym kann damit nicht nur zur Synthese von DHA aus DPA, sondern auch zur Synthese von speziellen Fettsäuren, die in der Natur nur begrenzt bis gar nicht vorkommen, verwendet werden. Beispiele solcher Fettsäuren sind 16:2 Δ4, Δ7 oder 16:3 Δ4, Δ7, Δ10, Δ13..

Dies unterscheidet vorteilhaft die gefundene Δ4-Desaturase neben der erhöhten Spezifität und Aktivität den Enzymen des Standes der Technik.

Da bisher beschriebene Δ4-Desaturasegene nur eine geringe Aktivität und Spezifität besitzen, bestand daher für die Erfindung weiterhin die Aufgabe spezifische Desaturase-Enzym für die Synthese mehrfach ungesättigter langkettiger Fettsäuren in den Samen von Ölsaaten einzubringen und die Produktion von unerwünschten Nebenprodukten zu vermeiden. Diese Aufgabe wurde durch die Klonierung der oben offenbarten Nukleinsäure gelöst.

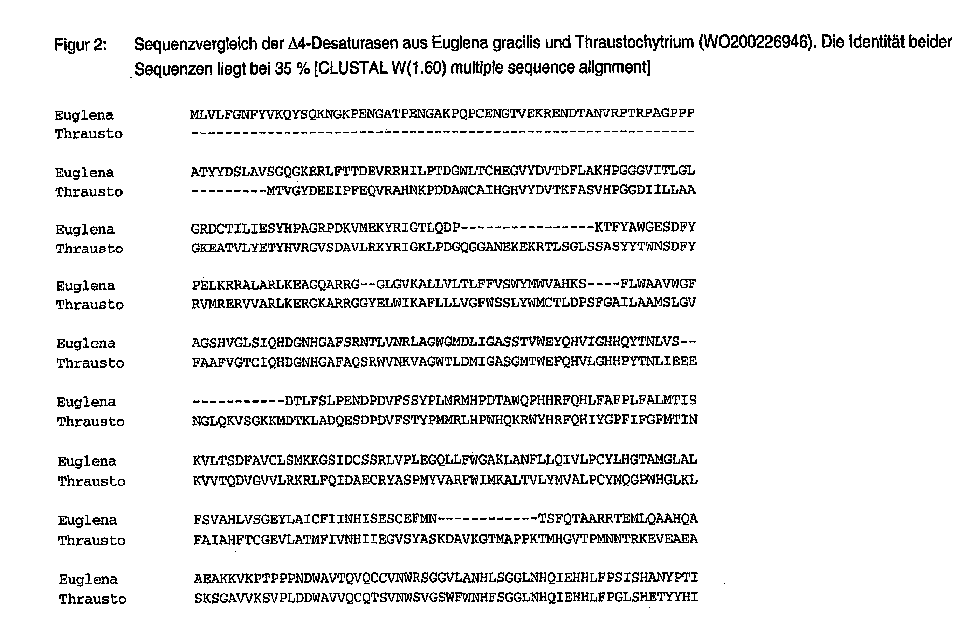

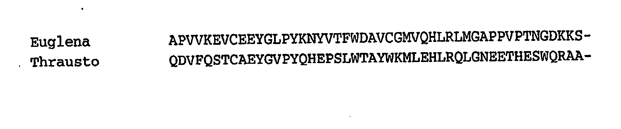

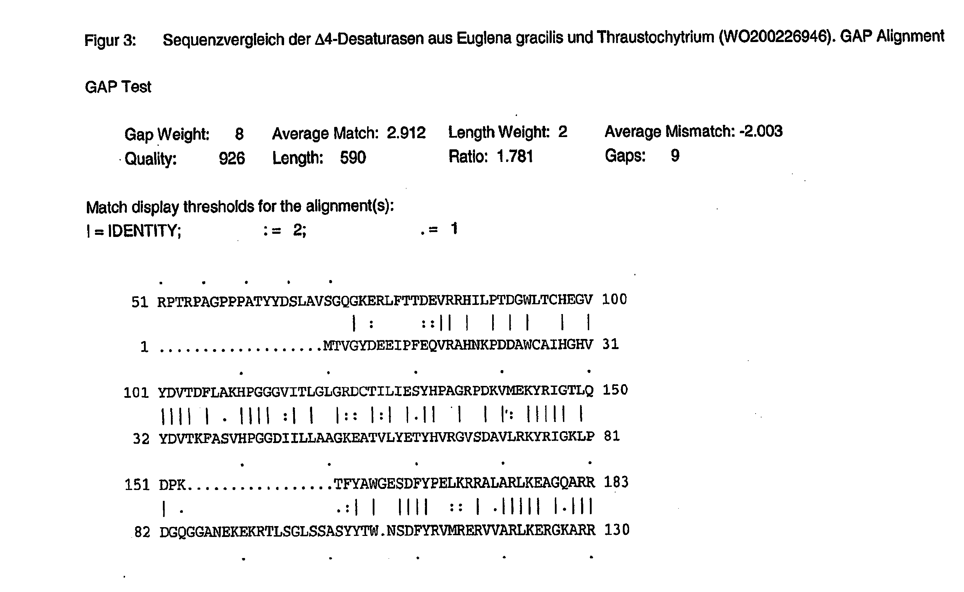

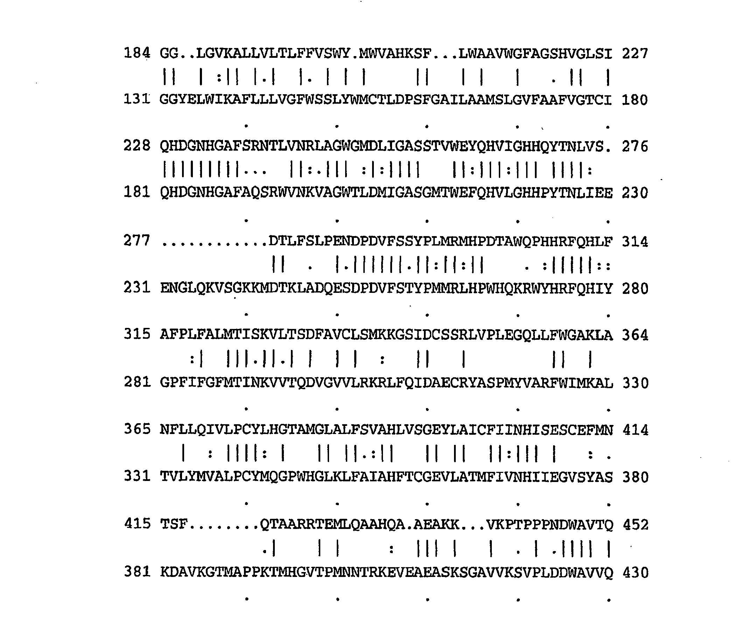

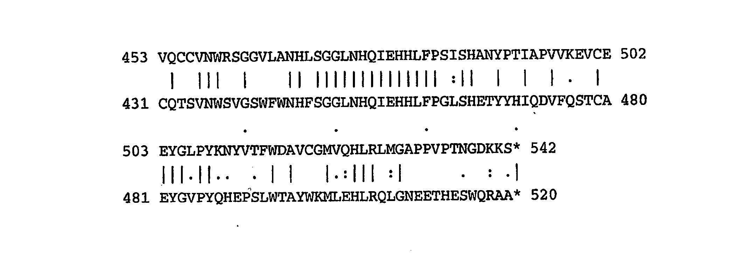

Die gefundene Δ-4-Desaturase unterscheidet sich von den bereits beschriebenen Δ4-Desaturasen durch wesentlich unterschiedliche Nukleotid- und Aminosäuresequenz. Die Euglena-Sequenz zeigt zu der Thraustochytrium-Sequenz (WO200226946) nur 35 % Ähnlichkeit auf Aminosäure-Ebene. In Fig. 2 ist ein Sequenzvergleich der gefundenen Euglena-Sequenz mit der Sequenz aus Thraustochytrium, in Fig. 3 ist das GAP-Alignment gezeigt.

Der Begriff "Δ-4-Desaturase" im Sinne der Erfindung umfasst Proteine, die an der Desaturierung von Fettsäuren vorteilhaft von Fettsäuren, die an Position 7 der Fettsäurekette eine Doppelbindung haben, teilnehmen, sowie ihre Homologen, Derivaten oder Analoga.

Bei einer weiteren Ausführungsform kodieren Derivate des erfindungsgemäßen Nukleinsäuremoleküls wieder gegeben in SEQ ID NO: 1 Proteine mit mindestens etwa 70 bis 80% bis 90% 90 bis 95 % und am stärksten bevorzugt mindestens etwa 96 %, 97 %, 98 %, 99 % oder mehr Homologie (= Identität) zur vollständigen Aminosäuresequenz der SEQ ID NO: 2. Die Homologie wurde über den gesamten Aminosäure- bzw. Nukleinsäuresequenzbereich berechnet. Für die Sequenzvergleiche wurde das Programm PileUp verwendet (J. Mol. Evolution., 25, 351-360, 1987, Higgins et al., CABIOS, 5 1989: 151-153) oder die Programme Gap und BestFit [Needleman and Wunsch (J. Mol. Biol. 48; 443-453 (1970) und Smith and Waterman (Adv. Appl. Math. 2; 482-489 (1981)], die im GCG Software-Packet [Genetics Computer Group, 575 Science Drive, Madison, Wisconsin, USA 53711 (1991)] enthalten sind. Die oben in Prozent angegebenen Sequenzhomologiewerte wurden mit dem Programm BestFit über den gesamten Sequenzbereich mit folgenden Einstellungen ermittelt: Gap Weight: 8, Length Weight: 2.

Die Erfindung umfasst zudem Nukleinsäuremoleküle, die sich von einer der in SEQ ID NO: 1 gezeigten Nukleotidsequenzen (und Teilen davon) aufgrund des degenerierten genetischen Codes unterscheiden und somit die gleiche Δ-4-Desaturase kodieren wie diejenige, die von der in SEQ ID NO: 1 gezeigten Nukleotidsequenz kodiert wird.

Zusätzlich zu der in SEQ ID NO: 1 gezeigten Δ-4-Desaturase-Nukleotidsequenz erkennt der Fachmann, dass DNA-Sequenzpolymorphismen, die zu Änderungen in den Aminosäuresequenzen der Δ-4-Desaturase führen, innerhalb einer Population existieren können. Diese genetischen Polymorphismen im Δ-4-Desaturase-Gen können zwischen Individuen innerhalb einer Population aufgrund von natürlicher Variation existieren. Diese natürlichen Varianten bewirken üblicherweise eine Varianz von 1 bis 5 % in der Nukleotidsequenz des Δ-4-Desaturase-Gens. Sämtliche und alle dieser Nukleotidvariationen und daraus resultierende Aminosäurepolymorphismen in der Δ-4-Desaturase, die das Ergebnis natürlicher Variation sind und die funktionelle Aktivität der Δ-4-Desaturase nicht verändern, sollen im Umfang der Erfindung enthalten sein.

Unter mehrfach ungesättigten Fettsäuren (PUFAS) sind im folgenden doppelt oder mehrfach ungesättigte Fettsäuren, die Doppelbindungen aufweisen, zu verstehen. Die Doppelbindungen können konjugiert oder nicht konjugiert sein.

Das erfindungsgemäße Enzym Δ-4-Desaturase führt vorteilhaft in Fettsäurereste von Glycerolipiden eine *cis*-Doppelbindung in Position C₄-C₅ ein (siehe SEQ ID NO: 1 und NO: 2). Das Enzym hat außerdem eine Δ-4-Desaturase-Aktivität, die vorteilhaft in Fettsäurereste von Glycerolipiden ausschließlich eine *cis*-Doppelbindung in Position C₄-C₅ einführt. Diese Aktivität hat auch das Enzym mit der in SEQ ID NO: 1 und NO: 2 genannten Sequenz, Bei den in SEQ ID NO: 1 und NO: 2 dargestellten Sequenzen handelt es sich um eine monofunktionelle Δ-4-Desaturase.

Die erfindungsgemäße Nukleinsäuresequenz (oder Fragmente davon können vorteilhaft zur Isolierung weiterer genomischer Sequenzen über Homologiescreening verwendet werden.

Die genannten Derivate lassen sich beispielsweise aus anderen Organismen eukaryontischen Organismen wie Pflanzen wie speziell Moosen, Dinoflagellaten oder Pilze isolieren.

Allelvarianten umfassen insbesondere funktionelle Varianten, die durch Deletion, Insertion oder Substitution von Nukleotiden aus der in SEQ ID NO: 1 dargestellten Sequenz erhältlich sind, wobei die enzymatische Aktivität der abgeleiteten synthetisierten Proteine erhalten bleibt.

Solche DNA-Sequenzen lassen sich ausgehend von den in SEQ ID NO: 1 beschriebenen DNA-Sequenz oder Teilen dieser Sequenzen, beispielsweise mit üblichen Hybridisierungsverfahren oder der PCR-Technik aus anderen Eukaryonten wie beispielsweise den oben genannt isolieren. Diese DNA-Sequenzen hybridisieren unter Standardbedingungen mit den genannten Sequenzen. Zur Hybridvisierung werden vorteilhaft kurze Oligonukleotide beispielsweise der konservierten Bereiche, die über Vergleiche mit anderen Desaturasegenen in dem Fachmann bekannter Weise ermittelt werden können, verwendet. Vorteilhaft werden die Histidin-Box-Sequenzen verwendet. Es können aber auch längere Fragmente der erfindungsgemäßen Nukleinsäuren oder die vollständigen Sequenzen für die Hybridisierung verwendet werden. Je nach der verwendeten Nukleinsäure: Oligonukleotid, längeres Fragment oder vollständige Sequenz oder je nachdem welche Nukleinsäureart DNA oder RNA für die Hybridisierung verwendet werden, variieren diese Standardbedingungen. So liegen beispielsweise die Schmelztemperaturen für DNA:DNA-Hybride ca. 10°C niedriger als die von DNA:RNA-Hybriden gleicher Länge.

Unter Standardbedingungen sind beispielsweise je nach Nukleinsäure Temperaturen zwischen 42 und 58°C in einer wässrigen Pufferlösung mit einer Konzentration zwischen 0,1 bis 5 x SSC (1 X SSC = 0,15 M NaCl, 15 mM Natriumcitrat, pH 7,2) oder zusätzlich in Gegenwart von 50 % Formamid wie beispielsweise 42°C in 5 x SSC, 50 % Formamid zu verstehen. Vorteilhafterweise liegen die Hybridisierungsbedingungen für DNA:DNA-Hybride bei 0,1 x SSC und Temperaturen zwischen etwa 20°C bis 45°C, bevorzugt zwischen etwa 30°C bis 45°C. Für DNA:RNA-Hybride liegen die Hybridisierungsbedingungen vorteilhaft bei 0,1 x SSC und Temperaturen zwischen etwa 30°C bis 55°C, bevorzugt zwischen etwa 45°C bis 55°C. Diese angegebenen Temperaturen für die Hybridisierung sind beispielhaft kalkulierte Schmelztemperaturwerte für eine Nukleinsäure mit einer Länge von ca. 100 Nukleotiden und einem G + C-Gehalt von 50 % in Abwesenheit von Formamid. Die experimentellen Bedingungen für die DNA-Hybridisierung sind in einschlägigen Lehrbüchern der Genetik wie beispielsweise Sambrook et al., "Molecular Cloning", Cold Spring Harbor Laboratory, 1989, beschrieben und lassen sich nach dem Fachmann bekannten Formeln beispielsweise abhängig von der Länge der Nukleinsäuren, der Art der Hybride oder dem G + C-Gehalt berechnen. Weitere Informationen zur Hybridisierung kann der Fachmann folgenden Lehrbüchern entnehmen: Ausubel et al. (eds), 1985, Current Protocols in Molecular Biology, John Wiley & Sons, New York; Hames and Higgins (eds), 1985, Nucleic Acids Hybridization: A Practical Approach, IRL Press at Oxford University Press, Oxford; Brown (ed), 1991, Essential Molecular Biology: A Practical Approach, IRL Press at Oxford University Press, Oxford.

Weiterhin sind unter Derivaten Homologe der Sequenz SEQ ID NO: 1 beispielsweise eukaryontische Homologe, verkürzte Sequenzen, Einzelstrang-DNA der codierenden und nichtcodierenden DNA-Sequenz oder RNA der codierenden und nichtcodierenden DNA-Sequenz zu verstehen.

Außerdem sind unter Homologen der Sequenz SEQ ID NO: 1 Derivate wie beispielsweise Promotorvarianten zu verstehen. Diese Varianten können durch ein oder mehrere Nukleotidaustausche, durch Insertion(en) und/oder Deletion(en) verändert sein, ohne dass aber die Funktionalität bzw. Wirksamkeit der Promotoren beeinträchtigt sind. Des weiteren können die Promotoren durch Veränderung ihrer Sequenz in ihrer Wirksamkeit erhöht oder komplett durch wirksamere Promotoren auch artfremder Organismen ausgetauscht werden.

Unter Derivaten sind auch vorteilhaft Varianten zu verstehen, deren Nukleotidsequenz im Bereich -1 bis -2000 vor dem Startkodon so verändert wurden, dass die Genexpression und/oder die Proteinexpression verändert, bevorzugt erhöht wird. Weiterhin sind unter Derivaten auch Varianten zu verstehen, die am 3'-Ende verändert wurden.

Unter Derivaten sind auch die Antisense-DNAs zu verstehen, die zur Hemmung der Proteinbiosynthese der erfindungsgemäßen Proteine verwendet werden können. Diese Antisense-DNAs gehören zu den erfindungsgemäßen nichtfunktionellen Derivaten, wie Derivate, die keine enzymatische Aktivität aufweisen. Weitere dem Fachmann bekannte Methoden der Herstellung von nichtfunktionellen Derivaten sind die sogenannte Cosuppression, die Verwendung von Ribozymen und introns. Ribozyme sind katalytische RNA-Moleküle mit Ribonukleaseaktivität, die Einzelstrang Nukleinsäuren wie mRNA, zu denen sie eine Komplementarität aufweisen, schneiden können. Dadurch können mit Hilfe dieser Ribozyme (Haselhoff and Gerlach, Nature, 334,1988: 585-591) mRNA-Transkripte katalytisch gespalten werden und so die Translation dieser mRNA unterdrückt werden. Derartige Ribozyme können speziell auf ihre Aufgaben hin zugeschnitten werden (US 4,987,071; US 5,116,742 und Bartel et al., Science 261, 1993: 1411-1418). Mit Hilfe der Antisense-DNA können dadurch Fettsäuren, Upide oder Öle mit einem erhöhten Anteil an gesättigten Fettsäuren hergestellt werden.

Die erfindungsgemäße Nukleinsäuresequenz, die für eine Δ-4-Desaturase kodiert, können synthetisch hergestellt oder natürlich gewonnen sein oder eine Mischung aus synthetischen und natürlichen DNA-Bestandteilen enthalten, sowie aus verschiedenen heterologen Δ-4-Desaturase-Genabschnitten verschiedener Organismen bestehen. Im allgemeinen werden synthetische Nukleotid-Sequenzen mit Codons erzeugt, die von den entsprechenden Wirtsorganismen beispielsweise Pflanzen bevorzugt werden. Dies führt in der Regel zu einer optimalen Expression der heterologen Gene. Diese von Pflanzen bevorzugten Codons können aus Codons mit der höchsten Proteinhäufigkeit bestimmt werden, die in den meisten interessanten Pflanzenspezies exprimiert werden. Ein Beispiel für Corynebacterium glutamicum ist gegeben in: Wada et al. (1992) Nucleic Acids Res. 20:2111-2118). Die Durchführung solcher Experimente sind mit Hilfe von Standardmethoden durchführbar und sind dem Fachmann auf dem Gebiet bekannt.

Funktionell äquivalente Sequenzen, die für das Δ-4-Desaturase-Gen kodieren, sind solche Derivate der erfindungsgemäßen Sequenz, welche trotz abweichender Nukleotidsequenz noch die gewünschten Funktionen, das heißt die enzymatische Aktivität und spezifische Selektivität der Proteine besitzen. Funktionelle Äquivalente umfassen somit natürlich vorkommende Varianten der hierin beschriebenen Sequenzen sowie künstliche, z.B. durch chemische Synthese erhaltene, an den Codon-Gebrauch einer Pflanze angepasste, künstliche Nukleotid-Sequenzen.

Außerdem sind artifizielle DNA-Sequenzen geeignet, solange sie, wie oben beschrieben, die gewünschte Eigenschaft beispielsweise der Erhöhung des Gehaltes von Δ-4-Doppelbindungen in Fettsäuren, Ölen oder Lipiden in der Pflanze durch Überexpression des Δ-4-Desaturase-Gens in Kulturpflanzen vermitteln. Solche artifiziellen DNA-Sequenzen können beispielsweise durch Rückübersetzung mittels Molecular Modelling konstruierter Proteine, Δ-4-Desaturase-Aktivität aufweisen oder durch in vitro-Selektion ermittelt werden. Mögliche Techniken zur in vitro-Evolution von DNA zur Veränderung bzw. Verbesserung der DNA-Sequenzen sind beschrieben bei Patten, P.A. et al., Current Opinion in Biotechnology 8, 724-733(1997) oder bei Moore, J.C. et al., Journal of Molecular Biology 272, 336-347 (1997). Besonders geeignet sind codierende DNA-Sequenzen, die durch Rückübersetzung einer Polypeptidsequenz gemäß der für die Wirtspflanze spezifischen Kodon-Nutzung erhalten werden. Die spezifische Kodon - Nutzung kann ein mit pflanzengenetischen Methoden vertrauter Fachmann durch Computerauswertungen anderer, bekannter Gene der zu transformierenden Pflanze leicht ermitteln.

Als weitere geeignete äquivalente Nukleinsäure-Sequenzen sind zu nennen Sequenzen, welche für Fusionsproteine kodieren, wobei Bestandteil des Fusionsproteins ein Δ-4-Desaturase-Polypeptid oder ein funktionell äquivalenter Teil davon ist. Der zweite Teil des Fusionsproteins kann z.B. ein weiteres Polypeptid mit enzymatischer Aktivität sein oder eine antigene Polypeptidsequenz mit deren Hilfe ein Nachweis auf Δ-4-Desaturase-Expression möglich ist (z.B. myc-tag oder his-tag). Bevorzugt handelt es sich dabei jedoch um eine regulative Proteinsequenz, wie z.B. ein Signalsequenz für das ER, das das Δ-4-Desaturase-Protein an den gewünschten Wirkort leitet.

Vorteilhaft stammen die erfindungsgemäßen isolierten Nukleinsäuresequenzen aus einer Pflanze wie einer monokotylen oder dikotylen Pflanze. Bevorzugt stammen die Nukleinsäuresequenzen aus der Klasse der Euglenophyceae wie den Ordnungen Eutreptiales, Euglenales, Rhabdomonadales, Sphenomonadales, Heteronematales oder Euglenamorphales besonders vorteilhaft stammen die Sequenzen aus der Gattung und Art Euglena gracilis, Astasia longa, Khawkinea quartana, Phacus smulkowskianus, Lepocinclis ovum, Lepocinclis ovata, Eutreptia viridis, Distigma proteus, Distigma curvatum, Rhabdomonas intermedia, Rhabdomonas gibba, Rhabdomonas spiralis, Gyropaigne lefevrei, Rhabdomonas incurva, Peranema trichophorum oder Petalomonas cantuscygni, ganz besonders vorteilhaft stammen sie aus Euglena gracilis.

Vorteilhaft können die Δ-4-Desaturase-Gene im erfindungsgemäßen Verfahren mit weiteren Genen der Fettsäurebiosynthese kombiniert werden. Beispiele für derartige Gene sind die Acyltransferasen, weitere Desaturasen oder Elongasen. Für die in vivo und speziell in vitro Synthese ist die Kombination mit z.B. NADH-Cytochrom B5 Reduktasen vorteilhaft, die Reduktionsäquivalente aufnehmen oder abgeben können.

Unter den erfindungsgemäßen Aminosäuresequenzen sind Proteine zu verstehen, die eine in der Sequenz SEQ ID NO: 2 dargestellten Aminosäuresequenz oder eine daraus durch Substitution, Inversion, Insertion oder Deletion von einem oder mehreren Aminosäureresten erhältliche Sequenz enthalten, wobei die enzymatische Aktivitäten des in SEQ ID NO: 2 dargestellten Proteins erhalten bleibt bzw. nicht wesentlich reduziert wird. Unter nicht wesentlich reduziert sind alle Enzyme zu verstehen, die noch mindestens 10 %, bevorzugt 20 %, besonders bevorzugt 30 % der enzymatischen Aktivität des Ausgangsenzyms aufweisen. Dabei können beispielsweise bestimmte Aminosäuren durch solche mit ähnlichen physikochemischen Eigenschaften (Raumerfüllung, Basizität, Hydrophobizität etc.) ersetzt werden. Beispielsweise werden Argininreste gegen Lysinreste, Valinreste gegen Isoleucinreste oder Asparaginsäurereste gegen Glutaminsäurereste ausgetauscht. Es können aber auch ein oder mehrere Aminosäuren in ihrer Reihenfolge vertauscht, hinzugefügt oder entfernt werden, oder es können mehrere dieser Maßnahmen miteinander kombiniert werden.

Unter Derivaten sind auch funktionelle Äquivalente zu verstehen, die insbesondere auch natürliche oder künstliche Mutationen einer ursprünglich isolierten für Δ-4-Desaturase codierende Sequenz beinhalten, welche weiterhin die gewünschte Funktion, das heißt deren enzymatische Aktivität und Substratselektivität nicht wesentlich reduziert ist, zeigen. Mutationen umfassen Substitutionen, Additionen, Deletionen, Vertauschungen oder Insertionen eines oder mehrerer Nukleotidreste. Somit werden beispielsweise auch solche Nukleotidsequenzen durch die vorliegende Erfindung mit umfasst, welche man durch Modifikation der Δ-4-Desaturase Nukleotidsequenz erhält. Ziel einer solchen Modifikation kann z.B. die weitere Eingrenzung der darin enthaltenen codierenden Sequenz oder z.B. auch die Einfügung weiterer Restriktionsenzym-Schnittstellen sein.

Funktionelle Äquivalente sind auch solche Varianten, deren Funktion, verglichen mit dem Ausgangsgen bzw. Genfragment, abgeschwächt (= nicht wesentlich reduziert) oder verstärkt ist (= Enzymaktivität stärker als die Aktivität des Ausgangsenzym, das heißt Aktivität ist höher als 100 %, bevorzugt höher als 110 %, besonders bevorzugt höher als 130 %).

Die Nukleinsäuresequenz kann dabei vorteilhaft beispielsweise eine DNA- oder cDNASequenz sein. Zur Insertion in eine erfindungsgemäßen Expressionskassette geeignete codierende Sequenzen sind beispielsweise solche, die für eine Δ-4-Desaturase mit den oben beschriebenen Sequenzen kodieren und die dem Wirt die Fähigkeit zur - Überproduktion von Fettsäuren, Ölen oder Lipiden mit Doppelbindungen in Δ-4-Position verleihen, besonders wenn dabei ω 3 Fettsäuren mit mindestens vier Doppelbindungen hergestellt werden. Diese Sequenzen können homologen oder heterologen Ursprungs sein.

Unter der erfindungsgemäßen Expressionskassette (= Nukleinsäurekonstrukt oder-fragment) sind die in SEQ ID NO: 1 genannte Sequenz, die sich als Ergebnis des genetischen Codes und/oder deren funktionellen oder nicht funktionellen Derivate zu verstehen, die mit einem oder mehreren Regulationssignalen vorteilhafterweise zur Erhöhung der Genexpression funktionell verknüpft wurden und welche die Expression der codierenden Sequenz in der Wirtszelle steuern. Diese regulatorischen Sequenzen sollen die gezielte Expression der Gene und der Proteinexpression ermöglichen. Dies kann beispielsweise je nach Wirtsorganismus bedeuten, dass das Gen erst nach Induktion exprimiert und/oder überexprimiert wird, oder dass es sofort exprimiert und/oder überexprimiert wird. Beispielsweise handelt es sich bei diesen regulatorischen Sequenzen um Sequenzen an die Induktoren oder Repressoren binden und so die Expression der Nukleinsäure regulieren. Zusätzlich zu diesen neuen Regulationssequenzen oder anstelle dieser Sequenzen kann die natürliche Regulation dieser Sequenzen vor den eigentlichen Strukturgenen noch vorhanden sein und gegebenenfalls genetisch verändert worden sein, so dass die natürliche Regulation ausgeschaltet und die Expression der Gene erhöht wurde. Das Genkonstrukt kann aber auch einfacher aufgebaut sein, das heißt es wurden keine zusätzlichen Regulationssignale vor die Nukleinsäuresequenz oder dessen Derivate inseriert und der natürliche Promotor mit seiner Regulation wurde nicht entfernt. Stattdessen wurde die natürliche Regulationssequenz so mutiert, dass keine Regulation mehr erfolgt und/oder die Genexpression gesteigert wird. Diese veränderten Promotoren können in Form von Teilsequenzen (= Promotor mit Teilen der erfindungsgemäßen Nukleinsäuresequenzen) auch allein vor das natürliche Gen zur Steigerung der Aktivität gebracht werden. Das Genkonstrukt kann außerdem vorteilhafterweise auch eine oder mehrere sogenannte "enhancer Sequenzen" funktionell verknüpft mit dem Promotor enthalten, die eine erhöhte Expression der Nukleinsäuresequenz ermöglichen. Auch am 3'-Ende der DNA-Sequenzen können zusätzliche vorteilhafte Sequenzen inseriert werden wie weitere regulatorische Elemente oder Terminatoren. Das Δ-4-Desaturase-Gen kann in einer oder mehreren Kopien in der Expressionskassette (= Genkonstrukt) enthalten sein.

Die regulatorischen Sequenzen bzw. Faktoren können dabei wie oben beschrieben vorzugsweise die Genexpression der eingeführten Gene positiv beeinflussen und dadurch erhöhen. So kann eine Verstärkung der regulatorischen Elemente vorteilhafterweise auf der Transkriptionsebene erfolgen, indem starke Transkriptionssignale wie Promotoren und/oder "Enhancer" verwendet werden. Daneben ist aber auch eine Verstärkung der Translation möglich, indem beispielsweise die Stabilität der mRNA verbessert wird.

Als Promotoren in der Expressionskassette sind grundsätzlich alle Promotoren geeignet, die die Expression von Fremdgenen in Organismen vorteilhaft in Pflanzen oder Pilzen steuern können. Vorzugsweise verwendet man insbesondere ein pflanzliche Promotoren oder Promotoren, die aus einem Pflanzenvirus entstammen. Vorteilhafte Regulationssequenzen für das erfindungsgemäße Verfahren sind beispielsweise in Promotoren wie cos-, tac-, trp-, tet-, trp-tet-, Ipp-, lac-, Ipp-lac-, lacl^{q-,} T7-, T5-, T3-, gal-, trc-, ara-, SP6-, λ-P_{R}- oder im λ-P_{L}-Promotor enthalten, die vorteilhafterweise in gram-negativen Bakterien Anwendung finden. Weitere vorteilhafte Regulationssequenzen sind beispielsweise in den gram-positiven Promotoren amy und SPO2, in den Hefe- oder Pilzpromotoren ADC1, MFα, AC, P-60, CYC1, GAPDH, TEF, rp28, ADH oder in den Pflanzenpromotoren wie CaMV/35S [Franck et al., Cell 21(1980) 285-294], SSU, OCS, lib4, STLS1, B33, nos (= Nopalin Synthase Promotor) oder im Ubiquitin-Promotor enthalten. Die Expressionskassette kann auch einen chemisch induzierbaren Promotor enthalten, durch den die Expression des exogenen Δ-4-Desaturase-Gens in den Organismen vorteilhaft in den Pflanzen zu einem bestimmten Zeitpunkt gesteuert werden kann. Derartige vorteilhafte Pflanzenpromotoren sind beispielsweise der PRP1-Promotor [Ward et al., Plant.Mol. Biol.22(1993), 361-366], ein durch Benzensulfonamid-induzierbarer (EP 388186), ein durch Tetrazyklin-induzierbarer (Gatz et al., (1992) Plant J. 2,397-404), ein durch Salizylsäure induzierbarer Promotor (WO 95/19443), ein durch Abscisinsäure-induzierbarer (EP335528) bzw. ein durch Ethanol- oder Cyclohexanon-induzierbarer (WO93/21334) Promotor. Weitere Pflanzenpromotoren sind beispielsweise der Promotor der cytosolischen FBPase aus Kartoffel, der ST-LSI Promotor aus Kartoffel (Stockhaus et al., EMBO J. 8 (1989) 2445-245), der Promotor der Phosphoribosylpyrophosphat Amidotransferase aus Glycine max (siehe auch Genbank Accession Nummer U87999) oder ein Nodien-spezifischen Promotor wie in EP 249676 können vorteilhaft verwandt werden. Vorteilhaft sind insbesonders solche pflanzliche Promotoren, die die Expression in Geweben oder Pflanzenteilen/-organen sicherstellen, in denen die Fettsäurebiosynthese bzw. dessen Vorstufen stattfindet wie beispielsweise im Endosperm oder im sich entwickelnden - Embryo. Insbesondere zu nennen sind vorteilhafte Promotoren, die eine samenspezifische Expression gewährleisten wie beispielsweise der USP-Promotor oder Derivate davon, der LEB4-Promotor, der Phaseolin-Promotor oder der Napin--Promotor. Der erfindungsgemäß aufgeführte und besonders vorteilhafte USP-Promotor oder dessen Derivate vermitteln in der Samenentwicklung eine sehr früh Genexpression (Baeumlein et al., Mol Gen Genet, 1991, 225 (3): 459-67). Weitere vorteilhafte samenspezifische Promotoren, die für monokotyle und dikotyle Pflanzen verwendet werden können, sind die für Dikotyle geeignete Promotoren wie ebenfalls beispielhaft ausgeführte Napingen-Promotor aus Raps (US5,608,152), der Oleosin-Promotor aus Arabidopsis (WO98/45461), der Phaseolin-Promotor aus Phaseolus vulgaris (US5,504,200), der Bce4-Promotor aus Brassica (WO91/13980) oder der Leguminosen B4-Promotor (LeB4, Baeumlein et al., Plant J., 2, 2, 1992: 233 - 239) oder für Monokotyle geeignete Promotoren wie die Promotoren die Promotoren des Ipt2- oder Ipt1-Gens aus Gerste (WO95/15389 und WO95/23230) oder die Promotoren des Gersten Hordein-Gens, des Reis Glutelin-Gens, des Reis Oryzin-Gens, des Reis Prolamin-Gens, des Weizen Gliadin-Gens, des Weizen Glutelin-Gens, des Mais Zein-Gens, des Hafer Glutelin-Gens, des Sorghum Kasirin-Gens oder des Roggen Secalin-Gens, die in WO99/16890 beschrieben werden.

Weiterhin sind insbesondere solche Promotoren bevorzugt, die die Expression in Geweben oder Pflanzenteilen sicherstellen, in denen beispielsweise die Biosynthese von Fettsäuren, Ölen und Lipiden bzw. deren Vorstufen stattfindet. Insbesondere zu nennen sind Promotoren, die eine samenspezifische Expression gewährleisten. Zu nennen sind der Promotor des Napin-Gens aus Raps (US 5,608,152), des USP-Promotor aus Vicia faba (USP = unbekanntes Samenprotein, Baeumlein et al., Mol Gen Genet, 1991, 225 (3): 459-67), des Oleosin-Gens aus Arabidopsis (WO98/45461), des Phaseolin-Promotors (US 5,504,200) oder der Promotor des Legumin B4-Gens (LeB4; Baeumlein et al.,1992, Plant Journal, 2 (2): 233-9). Weiterhin sind zu nennen Promotoren, wie der des Ipt2 oder Ipt1-Gens aus Gerste (WO95/15389 und WO95/23230), die in monokotylen Pflanzen samenspezifische Expression vermitteln.

In der Expressionskassette (= Genkonstrukt, Nukleinsäurekonstrukt) können wie oben beschrieben noch weitere Gene, die in die Organismen eingebracht werden sollen, enthalten sein. Diese Gene können unter getrennter Regulation oder unter der gleichen Regulationsregion wie das Δ-4-DESATURASE-Gen liegen. Bei diesen Genen handelt es sich beispielsweise um weitere Biosynthesegene vorteilhaft der Fettsäurebiosynthese wie Biosynthesegene des Fettsäure- oder Lipidstoffwechsels, die eine gesteigerte Synthese ermöglichen, ausgewählt aus der Gruppe Acyl-CoA-Dehydrogenase(n), Acyl-ACP[= acyl carrier protein]-Desaturase(n), Acyl-ACP-Thioesterase(n), Fettsäure-Acyl-Transferase(n), Acyl-CoA:Lysophospholipid-Acyltransferase(n), Fettsäure-Synthase(n), Fettsäure-Hydroxylase(n), Acetyl-Coenzym A-Carboxylase(n), Acyl-Coenzym A-Oxidase(n), Fettsäure-Desaturase(n), Fettsäure--Acetylenasen, Lipoxygenasen, Triacylglycerol-Lipasen, Allenoxid-Synthasen, Hydroperoxid-Lyasen oder Fettsäure-Elongase(n). Beispielsweise seien die Gene für die Δ-15-, Δ-12-, Δ-9-, Δ-6-, Δ-5-Desaturase, β-Ketoacyl-Reductasen, β-Ketoacyl-Synthasen, Elongasen oder die verschiedenen Hydroxylasen und Acyl-ACP-Thioesterasen genannt. Vorteilhaft werden Desaturase- und Elongasegene im Nukleinsäurekonstrukt verwendet. Besonders vorteilhaft werden Gene ausgewählt aus der Gruppe Δ-4-Desaturase-, Δ-5-Desaturase-, Δ-6-Desaturase-, Δ-8-Desatuase-, Δ-9-Desaturase-, Δ-12-Desaturase-, Δ-5-Elongase-, Δ-6-Elongase- oder Δ-9-Elongase im Konstrukt verwendet.

Prinzipiell können alle natürlichen Promotoren mit ihren Regulationssequenzen wie die oben genannten für die erfindungsgemäße Expressionskassette und das erfindungsgemäße Verfahren, wie unten beschrieben, verwendet werden. Darüber hinaus können auch synthetische Promotoren vorteilhaft verwendet werden.

Bei der Präparation einer Expressionskassette können verschiedene DNA-Fragmente manipuliert werden, um eine Nukleotid-Sequenz zu erhalten, die zweckmäßigerweise in der korrekten Richtung liest und die mit einem korrekten Leseraster ausgestattet ist. Für die Verbindung der DNA-Fragmente (= erfindungsgemäße Nukleinsäuren) miteinander können an die Fragmente Adaptoren oder Linker angesetzt werden.

Zweckmäßigerweise können die Promotor- und die Terminator-Regionen in Transkriptionsrichtung mit einem Linker oder Polylinker, der eine oder mehrere Restriktionsstel-len für die Insertion dieser Sequenz enthält, versehen werden. In der Regel hat der Linker 1 bis 10, meistens 1 bis 8, vorzugsweise 2 bis 6 Restriktionsstellen. Im allgemeinen hat der Linker innerhalb der regulatorischen Bereiche eine Größe von weniger als 100 bp, häufig weniger als 60 bp, mindestens jedoch 5 bp. Der Promotor kann sowohl nativ bzw. homolog als auch fremdartig bzw. heterolog zum Wirtsorganismus beispielsweise zur Wirtspflanze sein. Die Expressionskassette beinhaltet in der 5'-3'-Transkriptionsrichtung den Promotor, eine DNA-Sequenz, die für ein Δ-4-Desaturase-Gen codiert und eine Region für die transkriptionale Termination. Verschiedene Terminationsbereiche sind gegeneinander beliebig austauschbar.

Ferner können Manipulationen, die passende Restriktionsschnittstellen bereitstellen oder die überflüssige DNA oder Restriktionsschnittstellen entfernen, eingesetzt werden. Wo Insertionen, Deletionen oder Substitutionen wie z.B. Transitionen und Transversionen in Frage kommen, können *in vitro-*Mutagenese, -primerrepair-, Restriktion oder Ligation verwendet werden. Bei geeigneten Manipulationen, wie z.B. Restriktion, -chewing-back- oder Auffüllen von Überhängen für -bluntends-, können komplementäre Enden der Fragmente für die Ligation zur Verfügung gestellt werden.

Von Bedeutung für eine vorteilhafte hohe Expression kann u.a. das Anhängen des spezifischen ER-Retentionssignals SEKDEL sein (Schouten, A. et al., Plant Mol. Biol. 30 (1996), 781-792), die durchschnittliche Expressionshöhe wird damit verdreifacht bis vervierfacht. Es können auch andere Retentionssignale, die natürlicherweise bei im ER lokalisierten pflanzlichen und tierischen Proteinen vorkommen, für den Aufbau der Kassette eingesetzt werden.

Bevorzugte Polyadenylierungssignale sind pflanzliche Polyadenylierungssignale, vorzugsweise solche, die im wesentlichen T-DNA-Polyadenylierungssignale aus Agrobacterium tumefaciens, insbesondere des Gens 3 derT-DNA (Octopin Synthase) des Ti-Plasmids pTiACH5 entsprechen (Gielen et al., EMBO J.3 (1984), 835 ff) oder entsprechende funktionelle Äquivalente.

Die Herstellung einer Expressionskassette erfolgt durch Fusion eines geeigneten Promotors mit einer geeigneten Δ-4-Desaturase-DNA-Sequenz sowie einem Polyadenylierungssignal nach gängigen Rekombinations- und Klonierungstechniken, wie sie beispielsweise in T. Maniatis, E.F. Fritsch und J. Sambrook, Molecular Cloning: A Laboratory Manual, Cold Spring Harbor Laboratory, Cold Spring Harbor, NY (1989) sowie in T.J. Silhavy, M.L Berman und L.W. Enquist, Experiments with Gene Fusions, Cold Spring Harbor Laboratory, Cold Spring Harbor, NY (1984) und in Ausubel, F.M. et al., Current Protocols in Molecular Biology, Greene Publishing Assoc. and Wiley-Interscience (1987) beschrieben werden.

Bei der Präparation einer Expressionskassette können verschiedene DNA-Fragmente manipuliert werden, um eine Nukleotid-Sequenz zu erhalten, die zweckmäßigerweise in der korrekten Richtung liest und die mit einem korrekten Leseraster ausgestattet ist. Für die Verbindung der DNA-Fragmente miteinander können an die Fragmente Adaptoren oder Linker angesetzt werden.

Zweckmäßigerweise können die Promotor- und die Terminator-Regionen in Transkriptionsrichtung mit einem Linker oder Polylinker, der eine oder mehrere Restriktionsstellen für die Insertion dieser Sequenz enthält, versehen werden. In der Regel hat der Linker 1 bis 10, meistens 1 bis 8, vorzugsweise 2 bis 6 Restriktionsstellen. Im allgemeinen hat der Linker innerhalb der regulatorischen Bereiche eine Größe von weniger als 100 bp, häufig weniger als 60 bp, mindestens jedoch 5 bp. Der Promotor kann sowohl nativ bzw. homolog als auch fremdartig bzw. heterolog zur Wirtspflanze sein. Die Expressionskassette beinhaltet in der 5'-3'-Transkriptionsrichtung den Promotor, eine DNA-Sequenz die für ein Δ-4-Desaturase-Gen codiert und eine Region für die transkriptionale Termination. Verschiedene Terminationsbereiche sind gegeneinander beliebig austauschbar.

Bei der Präparation einer Expressionskassette können verschiedene DNA-Fragmente manipuliert werden, um eine Nukleotid-Sequenz zu erhalten, die zweckmäßigerweise in der korrekten Richtung liest und die mit einem korrekten Leseraster ausgestattet ist. Für die Verbindung der DNA-Fragmente miteinander können an die Fragmente Adaptoren oder Linker angesetzt werden.

Zweckmäßigerweise können die Promotor- und die Terminator-Regionen in Transkriptionsrichtung mit einem Linker oder Polylinker, der eine oder mehrere Restriktionsstel-len für die Insertion dieser Sequenz enthält, versehen werden. In der Regel hat der Linker 1 bis 10, meistens 1 bis 8, vorzugsweise 2 bis 6 Restriktionsstellen. Im allgemeinen hat der Linker innerhalb der regulatorischen Bereiche eine Größe von weniger als 100 bp, häufig weniger als 60 bp, mindestens jedoch 5 bp. Der Promotor kann sowohl nativ bzw. homolog als auch fremdartig bzw. heterolog zur Wirtspflanze sein. Die Expressionskassette beinhaltet in der 5'-3'-Transkriptionsrichtung den Promotor, eine DNA-Sequenz die für ein Δ-4-Desaturase-Gen codiert und eine Region für die transkriptionale Termination. Verschiedene Terminationsbereiche sind gegeneinander beliebig austauschbar.

Die DNA Sequenzen codierend für zwei Δ-4-Desaturasen aus Euglena gracilis beinhaltet alle Sequenzmerkmale, die notwendig sind, um eine dem Ort der Fettsäure-, Lipid- oder Ölbiosynthese korrekte Lokalisation zu erreichen. Daher sind keine weiteren Targetingsequenzen per se notwendig. Allerdings kann eine solche Lokalisation wünschenswert und vorteilhaft sein und daher künstlich verändert oder verstärkt werden, so dass auch solche Fusionskonstrukte eine bevorzugte vorteilhafte Ausführungsform der Erfindung sind.

Insbesondere bevorzugt sind Sequenzen, die ein Targeting in Plastiden gewährleisten. Unter bestimmten Umständen kann auch ein Targeting in andere Kompartimente (referiert: Kermode, Crit. Rev. Plant Sci. 15,4 (1996), 285-423) z.B. in die Vakuole, in das Mitochondrium, in das Endoplasmatische Retikulum (ER), Peroxisomen, Lipidkörper oder durch ein Fehlen entsprechender operativer Sequenzen ein Verbleib im Kompartiment des Entstehens, dem Zytosol, wünschenswert sein.

Vorteilhafterweise werden die erfindungsgemäßen Nukleinsäuresequenzen zusammen mit mindestens einem Reportergen in eine Expressionskassette kloniert, die in den Organismus über einen Vektor oder direkt in das Genom eingebracht wird. Dieses Reportergen sollte eine leichte Detektierbarkeit über einen Wachstums-, Fluoreszenz-, Chemo-, Biolumineszenz- oder Resistenzassay oder über eine photometrische Messung ermöglichen. Beispielhaft seien als Reportergene Antibiotika- oder Herbizidresistenzgenre, Hydrolasegene, Fluoreszenzproteingene, Biolumineszenzgene, Zucker- oder Nukleotidstoffwechselgene oder Biosynthesegene wie das Ura3-Gen, das IIv2-Gen, das Luciferasegen, das α-Galactosidasegen, das gfp-Gen, das 2-Desoxyglucose-6-phosphat-Phosphatasegen, das β-Glucuronidase-Gen, β-Lactamasegen, das Neomycinphosphotransferasegen, das Hygromycinphosphotransferasegen oder das BASTA (= Gluphosinatresistenz)-Gen genannt. Diese Gene ermöglichen eine leichte Messbarkeit und Quantifizierbarkeit der Transcriptionsaktivität und damit der Expression der Gene. Damit lassen sich Genomstellen identifizieren, die eine unterschiedliche Produktivität zeigen.

Gemäß einer bevorzugten Ausführungsform umfasst eine Expressionskassette stromaufwärts, d.h. am 5'-Ende der codierenden Sequenz, einen Promotor und stromabwärts, d.h. am 3'-Ende, ein Polyadenylierungssignal und gegebenenfalls weitere regulatorische Elemente, welche mit der dazwischenliegenden codierenden Sequenz für die Δ-4-Desaturase und/oder Δ-4-Desaturase DNA Sequenz operativ verknüpft sind. Unter einer operativen Verknüpfung versteht man die sequenzielle Anordnung von Promotor, codierender Sequenz, Terminator und ggf. weiterer regulativer Elemente derart, dass jedes der regulativen Elemente seine Funktion bei der Expression der codierenden Sequenz bestimmungsgemäß erfüllen kann. Die zur operativen Verknüpfung bevorzugten Sequenzen sind Targeting-Sequenzen zur Gewährleistung der subzellulären Lokalisation in Plastiden. Aber auch Targeting-Sequenzen zur Gewährleistung der subzellulären Lokalisation im Mitochondrium, im Endoplasmatischen Retikulum (= ER), im Zellkern, in Ölkörperchen oder anderen Kompartimenten sind bei Bedarf einsetzbar sowie Translationsverstärker wie die 5'-Führungssequenz aus dem Tabak-Mosaik-Virus (Gallie et al., Nucl. Acids Res. 15 (1987), 8693 -8711).

Eine Expressionskassette kann beispielsweise einen konstitutiven Promotor (bevorzugt den USP- oder Napin-Promotor), das zu exprimierende Gen und das ER-Retentionssignal enthalten. Als ER-Retentionssignal wird bevorzugt die Aminosäuresequenz KDEL (Lysin, Asparaginsäure, Glutaminsäure, Leucin) verwendet.

Die Expressionskassette wird zur Expression in einem prokaryontischen oder eukaryontischen Wirtsorganismus beispielsweise einem Mikroorganismus wie einem Pilz oder einer Pflanze vorteilhafterweise in einen Vektor wie beispielsweise einem Plasmid, einem Phagen oder sonstiger DNA inseriert, der eine optimale Expression der Gene im Wirtsorganismus ermöglicht. Geeignete Plasmide sind beispielsweise in E. coli pLG338, pACYC184, pBR-Serie wie z.B. pBR322, pUC-Serie wie pUC18 oder pUC19, M113mp-Serie, pKC30, pRep4, pHS1, pHS2, pPLc236, pMBL24, pLG200, pUR290, pIN-III¹¹³-B1, λgt11 oder pBdCl, in Streptomyces pIJ101, pIJ364, pIJ702 oder pIJ361, in Bacillus pUB110, pC194 oder pBD214, in Corynebacterium pSA77 oder pAJ667, in Pilzen pALS1, pIL2 oder pBB116, weitere vorteilhafte Pilzvektoren werden von Romanos, M.A. et al., [(1992) "Foreign gene expression in yeast: a review", Yeast 8: 423-488] und von van den Hondel, C.A.M.J.J. et al. [(1991) "Heterologous gene expression in filamentous fungi] sowie in More Gene Manipulations in Fungi [J.W. Bennet & LL. Lasure, eds., p. 396-428: Academic Press: San Diego] und in "Gene transfer systems and vector development for filamentous fungi" [van den Hondel, C.A.M.J.J. & Punt, P.J. (1991) in: Applied Molecular Genetics of Fungi, Peberdy, J.F. et al., eds., p. 1-28, Cambridge University Press: Cambridge] beschrieben. Vorteilhafte Hefepromotoren sind beispielsweise 2∝M, pAG-1, YEp6, YEp13 oder pEMBLYe23. Beispiele für Algen- oder Pflanzenpromotoren sind pLGV23, pGHIac⁺, pBIN19, pAK2004, pVKH oder pDH51 (siehe Schmidt, R. and Willmitzer, L., 1988). Die oben genannten Vektoren oder Derivate der vorstehend genannten Vektoren stellen eine kleine Auswahl der möglichen Plasmide dar. Weitere Plasmide sind dem Fachmann wohl bekannt und können beispielsweise aus dem Buch Cloning Vectors (Eds. Pouwels P.H. et al. Elsevier, Amsterdam-New York-Oxford, 1985, ISBN 0 444 904018) entnommen werden. Geeignete pflanzliche Vektoren werden unter anderem in "Methods in Plant Molecular Biology and Biotechnology" (CRC Press), Kap. 6/7, S.71-119 beschrieben. Vorteilhafte Vektoren sind sog. shuttle-Vektoren oder binäre Vektoren, die in E. coli und Agrobacterium replizieren.

Unter Vektoren sind außer Plasmiden auch alle anderen dem Fachmann bekannten Vektoren wie beispielsweise Phagen, Viren wie SV40, CMV, Baculovirus, Adenovirus, Transposons, IS-Elemente, Phasmide, Phagemide, Cosmide, lineare oder zirkuläre DNA zu verstehen. Diese Vektoren können autonom im Wirtsorganismus repliziert oder chromosomal repliziert werden bevorzugt ist eine chromosomale Replikation.

In einer weiteren Ausgestaltungsform des Vektors kann die erfindungsgemäße Expressionskassette auch vorteilhafterweise in Form einer linearen DNA in die Organismen eingeführt werden und über heterologe oder homologe Rekombination in das Genom des Wirtsorganismus integriert werden. Diese lineare DNA kann aus einem linearisierten Plasmid oder nur aus der Expressionskassette als Vektor oder den erfindungsgemäßen Nukleinsäuresequenzen bestehen.

In einer weiteren vorteilhaften Ausführungsform kann die erfindungsgemäße Nukleinsäuresequenz auch alleine in einen Organismus eingebracht werden.

Sollen neben der erfindungsgemäßen Nukleinsäuresequenz weitere Gene in den Organismus eingeführt werden, so können alle zusammen mit einem Reportergen in einem einzigen Vektor oder jedes einzelne Gen mit einem Reportergen in je einem Vektor in den Organismus eingebracht werden, wobei die verschiedenen Vektoren gleichzeitig oder sukzessive eingebracht werden können.

Der Vektor enthält vorteilhaft mindestens eine Kopie der erfindungsgemäßen Nukleinsäuresequenzen und/oder der erfindungsgemäßen Expressionskassette.

Beispielhaft kann die pflanzliche Expressionskassette in den Transformationsvektor pRT ((a) Toepfer et al., 1993, Methods Enzymol., 217: 66-78; (b) Toepfer et al. 1987, Nucl. Acids. Res. 15: 5890 ff.) eingebaut werden.

Alternativ kann ein rekombinanter Vektor (= Expressionsvektor) auch in-vitro transkribiert und translatiert werden, z.B. durch Nutzung des T7 Promotors und der T7 RNA Polymerase.

In Prokaryoten verwendete Expressionsvektoren nutzen häufig induzierbare Systeme mit und ohne Fusionsproteinen bzw. Fusionsobligopeptiden, wobei diese Fusionen sowohl Kn-Terminal als auch C-terminal oder anderen nutzbaren Domänen eines Proteins erfolgen können. Solche Fusionsvektoren dienen in der Regel dazu: i.) die Expressionsrate der RNA zu erhöhen ii.) die erzielbare Proteinsyntheserate zu erhöhen, iii.) die Löslichkeit des Proteins zu erhöhen, iv.) oder die Reinigung durch einen für die Affinitätschromatographie nutzbare Bindesequenz zu vereinfachen. Häufig werden auch proteolytische Spaltstellen über Fusionsproteine eingeführt, was die Abspaltung eines Teils des Fusionsproteins auch der Reinigung ermöglicht Solche Erkennungssequenzen für Proteasen erkennen sind z.B. Faktor Xa, Thrombin und Enterokinase.

Typische vorteilhafte Fusions- und Expressionsvektoren sind pGEX [Pharmacia Biotech Inc; Smith, D.B. and Johnson, K.S. (1988) Gene 67: 31-40], pMAL (New England Biolabs, Beverly, MA) and pRIT5 (Pharmacia, Piscataway, NJ) welches Glutathion S-transferase beinhaltet (GST), Maltose Bindeprotein, oder Protein A.

Weitere Beispiele für E. coli Expressionsvektoren sind pTrc [Amann et al., (1988) Gene 69:301-315] und pET Vektoren [Studier et al., Gene Expression Technology: Methods in Enzymology 185, Academic Press, San Diego, California (1990) 60-89; Stratagene, Amsterdam, Niederlande].

Weitere vorteilhafte Vektoren zur Verwendung in Hefe sind pYepSec1 (Baldari, et al., (1987) Embo J. 6:229-234), pMFa (Kurjan and Herskowitz, (1982) Cell 30:933-943), pJRY88 (Schultz et al., (1987) Gene 54:113-123), and pYES-Derivate (Invitrogen Corporation, San Diego, CA). Vektoren für die Nutzung in filamentösen Pilzen sind beschrieben in: van den Hondel, C.A.M.J.J. & Punt, P.J. (1991) "Gene transfer systems and vector development for filamentous fungi, in: Applied Molecular Genetics of Fungi, J.F. Peberdy, et al., eds., p. 1-28, Cambridge University Press: Cambridge.

Alternativ können auch vorteilhaft Insektenzellexpressionsvektoren genutzt werden z.B. für die Expression in Sf 9 Zellen. Dies sind z.B. die Vektoren der pAc Serie (Smith et al. (1983) Mol. Cell Biol. 3:2156-2165) und der pVL series (Lucklow and Summers (1989) Virology 170:31-39).

Des weiteren können zur Genexpression vorteilhaft Pflanzenzellen oder Algenzellen genutzt werden. Beispiele für Pflanzenexpressionsvektoren finden sich in Becker, D., et al. (1992) "New plant binary vectors with selectable markers located proximal to the left border", Plant Mol. Biol. 20: 1195-1197 oder in Bevan, M.W. (1984) "Binary Agrobacterium vectors for plant transformation", Nucl. Acid. Res. 12: 8711-8721.

Weiterhin können die erfindungsgemäßen Nukleinsäuresequenzen in Säugerzellen exprimiert werden. Beispiel für entsprechende Expressionsvektoren sind pCDM8 und pMT2PC genannt in: Seed, B. (1987) Nature 329:840 oder Kaufman et al. (1987) EMBO J. 6:187-195). Dabei sind vorzugsweise zu nutzende Promotoren viralen Ursprungs wie z.B. Promotoren des Polyoma, Adenovirus 2, Cytomegalovirus oder Simian Virus 40. Weitere prokaryotische und eukaryotische Expressionssysteme sind genannt in Kapitel 16 und 17 in Sambrook et al., Molecular Cloning: A Laboratory Manual. 2nd, ed., Cold Spring Harbor Laboratory, Cold Spring Harbor Laboratory Press, Cold Spring Harbor, NY, 1989.

Das Einbringen der erfindungsgemäßen Nukleinsäuren, der Expressionskassette oder des Vektors in Organismen beispielsweise in Pflanzen kann prinzipiell nach allen dem Fachmann bekannten Methoden erfolgen.

Für Mikroorganismen kann der Fachmann entsprechende Methoden den Lehrbüchern von Sambrook, J. et al. (1989) Molecular cloning: A laboratory manual, Cold Spring Harbor Laboratory Press, von F.M. Ausubel et al. (1994) Current protocols in molecular biology, John Wiley and Sons, von D.M. Glover et al., DNA Cloning Vol. 1, (1995), IRL Press (ISBN 019-963476-9), von Kaiser et al. (1994) Methods in Yeast Genetics, Cold Spring Habor Laboratory Press oder Guthrie et al. Guide to Yeast Genetics and Molecular Biology, Methods in Enzymology, 1994, Academic Press entnehmen.

Die Übertragung von Fremdgenen in das Genom einer Pflanze wird als Transformation bezeichnet. Es werden dabei die beschriebenen Methoden zur Transformation und Regeneration von Pflanzen aus Pflanzengeweben oder Pflanzenzellen zur transienten oder stabilen Transformation genutzt. Geeignete Methoden sind die Protoplastentransformation durch Polyethylenglykol-induzierte DNA-Aufnahme, das biolistische Verfahren mit der Genkanone - die sogenannte particle bombardment Methode, die Elektroporation, die Inkubation trockener Embryonen in DNA-haltiger Lösung, die Mikroinjektion und der durch Agrobacterium vermittelte Gentransfer. Die genannten Verfahren sind beispielsweise in B. Jenes et al., Techniques for Gene Transfer, in: Transgenic Plants, Vol. 1, Engineering and Utilization, herausgegeben von S.D. Kung und R. Wu, Academic Press (1993) 128-143 sowie in Potrykus Annu. Rev. Plant Physiol. Plant Molec. Biol. 42 (1991) 205-225) beschrieben. Vorzugsweise wird das zu exprimierende Konstrukt in einen Vektor kloniert, der geeignet ist, Agrobacterium tumefaciens zu transformieren, beispielsweise pBin19 (Bevan et al., Nucl. Acids Res. 12 (1984) 8711). Mit einem solchen Vektor transformierte Agrobakterien können dann in bekannter Weise zur Transformation von Pflanzen, insbesondere von Kulturpflanzen, wie z.B. von Tabakpflanzen, verwendet werden, indem beispielsweise verwundete Blätter oder Blattstücke in einer Agrobakterienlösung gebadet und anschließend in geeigneten Medien kultiviert werden. Die Transformation von Pflanzen mit Agro-bacterium tumefaciens wird beispielsweise von Höfgen und Willmitzer in Nucl. Acid Res. (1988) 16, 9877 beschrieben oder ist unter anderem bekannt aus F.F. White, Vectors for Gene Transfer in Higher Plants; in Transgenic Plants, Vol. 1, Engineering and Utilization, herausgegeben von S.D. Kung und R. Wu, Academic Press, 1993, S. 15-38.

Mit einem erfindungsgemäßen Expressionsvektor transformierte Agrobakterien können ebenfalls in bekannter Weise zur Transformation von Pflanzen wie Testpflanzen wie Arabidopsis oder Kulturpflanzen wie Getreide, Mais, Hafer, Roggen, Gerste, Weizen, Soja, Reis, Baumwolle, Zuckerrübe, Canola, Sonnenblume, Flachs, Hanf, Kartoffel, Tabak, Tomate, Karotte, Paprika, Raps, Tapioka, Maniok, Pfeilwurz, Tagetes, Alfalfa, Salat und den verschiedenen Baum-, Nuss- und Weinspezies, insbesondere von Öl-haltigen Kulturpflanzen, wie Soja, Erdnuss, Rizinus, Sonnenblume, Mais, Baumwolle, Flachs, Raps, Kokosnuss, Ölpalme, Färbersaflor (Carthamus tinctorius) oder Kakaobohne verwendet werden, z.B. indem verwundete Blätter oder Blattstücke in einer Agrobakterienlösung gebadet und anschließend in geeigneten Medien kultiviert werden. Besonders zur Herstellung von PUFAs, beispielsweise Stearidonsäure, Eicosapentaensäure und Docosahexaensäure eignen sich Borage oder Primulaceen. Besonders vorteilhaft eignet sich Lein zur Herstellung von PUFAS mit dem erfindungsgemäßen Nukleinsäuresequenzen vorteilhaft in Kombination mit weiteren Desaturasen und Elongasen.

Die genetisch veränderten Pflanzenzellen können über alle dem Fachmann bekannten Methoden regeneriert werden. Entsprechende Methoden können den oben genannten Schriften von S.D. Kung und R. Wu, Potrykus oder Höfgen und Willmitzer entnommen werden.

Als transgene Organismen bzw. Wirtsorganismen für die erfindungsgemäße Nukleinsäure, die Expressionskassette oder den Vektor eignen sich prinzipiell vorteilhaft alle nicht-humanen Organismen, die in der Lage sind Fettsäuren speziell ungesättigte Fettsäuren zu synthetisieren bzw. für die Expression rekombinanter Gene geeignet sind wie Mikrororganismen, nicht-humane Tiere oder Pflanzen. Beispielhaft seien Pflanzen wie Arabidopsis, Asteraceae wie Calendula oder Kulturpflanzen wie Soja, Erdnuss, Rizinus, Sonnenblume, Mais, Baumwolle, Flachs, Raps, Kokosnuss, Ölpal-me, Färbersaflor (Carthamus tinctorius) oder Kakaobohne, Mikroorganismen wie Pilze beispielsweise die Gattung Mortierella, Saprolegnia oder Pythium, Bakterien wie die Gattung Escherichia, Hefen wie die Gattung Saccharomyces, Cyanobakterien, Ciliaten, Algen oder Protozoen wie Dinoflagellaten wie Crypthecodinium genannt. Bevorzugt werden Organismen, die natürlicherweise Öle in größeren Mengen synthetisieren können wie Pilze wie Mortierella alpina, Pythium insidiosum oder Pflanzen wie Soja, Raps, Kokosnuss, Ölpalme, Färbersaflor, Rizinus, Calendula, Erdnuss, Kakaobohne oder Sonnenblume oder Hefen wie Saccharomyces cerevisiae, besonders bevorzugt werden Soja, Raps, Sonnenblume, Lein, Calendula oder Saccharomyces cerevisiae. Prinzipiell sind als Wirtsorganismen auch nicht-humane transgene Tiere geeignet beispielsweise C. elegans.

Unter transgenen Organismus bzw. transgener Pflanze im Sinne der Erfindung ist zu verstehen, dass die im Verfahren verwendeten Nukleinsäuren nicht an ihrer natürlichen Stelle im Genom eines Organismus sind, dabei können die Nukleinsäuren homolog oder heterolog exprimiert werden. Transgen bedeutet aber auch wie genannt, dass die erfindungsgemäßen Nukleinsäuren an ihrem natürlichen Platz im Genom eines Organismus sind, dass jedoch die Sequenz gegenüber der natürlichen Sequenz verändert wurde und/oder das die Regulationssequenzen, der natürlichen Sequenzen verändert wurden. Bevorzugt ist unter transgen die Expression der erfindungsgemäßen Nukleinsäuren an nicht natürlicher Stelle im Genom zu verstehen, das heißt eine homologe oder bevorzugt heterologe Expression der Nukleinsäuren liegt vor. Bevorzugte transgene Organismen sind Pilze wie Mortierella oder Pflanzen sind die Öl-fruchtpflanzen.

"Transgen" meint damit beispielsweise bezüglich einer Nukleinsäuresequenz, einer Expressionskassette oder einem Vektor enthaltend eine Nukleinsäuresequenz, die für die Δ-4-Desaturase oder deren Derivate codiert, oder einem Organismus transformiert mit dieser Nukleinsäuresequenz, einer Expressionskassette oder einem Vektor alle solche durch gentechnische Methoden zustandegekommene Konstruktionen, in denen sich entweder
a) die Δ-4-Desaturase-Nukleinsäuresequenz, oder
b) eine mit der Δ-4-Desaturase-Nukleinsäuresequenz funktionell verknüpfte genetische Kontrollsequenz, zum Beispiel ein Promotor, oder
c) (a) und (b)
sich nicht in ihrer natürlichen, genetischen Umgebung befinden oder durch gentechnische Methoden modifiziert wurden, wobei die Modifikation beispielhaft eine Substitutionen, Additionen, Deletionen, Inversion oder Insertionen eines oder mehrerer Nukleotidreste sein kann. Natürliche genetische Umgebung meint den natürlichen chromosomalen Locus in dem Herkunftsorganismus oder das Vorliegen in einer genomischen Bibliothek. Im Fall einer genomischen Bibliothek ist die natürliche, genetische Umgebung der Nukleinsäuresequenz bevorzugt zumindest noch teilweise erhalten. Die Umgebung flankiert die Nukleinsäuresequenz zumindest an einer Seite und hat eine Sequenzlänge von mindestens 50 bp, bevorzugt mindestens 500 bp, besonders bevorzugt mindestens 1000 bp, ganz besonders bevorzugt mindestens 5000 bp.

Nutzbare Wirtszellen sind weiterhin genannt in: Goeddel, Gene Expression Technology: Methods in Enzymology 185, Academic Press, San Diego, CA (1990).

Verwendbare Expressionsstämme z.B. solche, die eine geringere Proteaseaktivität aufweisen sind beschrieben in: Gottesman, S., Gene Expression Technology: Methods in Enzymology 185, Academic Press, San Diego, California (1990) 119-128.

Ein weiterer Gegenstand der Erfindung betrifft die Verwendung einer Expressionskassette enthaltend DNA-Sequenzen codierend für ein Δ-4-Desaturase-Gen oder mit diesen hybridisierende DNA-Sequenzen zur Transformation von Pflanzenzellen, - geweben oder Pflanzenteilen. Ziel der Verwendung ist die Erhöhung des Gehaltes an Fettsäuren, Ölen oder Lipiden mit erhöhtem Gehalt an und Doppelbindungen in Δ-4-Position.

Dabei kann je nach Wahl des Promotors die Expression des Δ-4-Desaturase-Gens spezifisch in den Blättern, in den Samen, den Knollen oder anderen Teilen der Pflanze erfolgen. Solche Fettsäuren, Öle oder Lipide mit Δ-4-Doppeltiindungen überproduzierenden transgenen Pflanzen, deren Vermehrungsgut, sowie deren Pflanzenzellen, - gewebe oder -teile, sind ein weiterer Gegenstand der vorliegenden Erfindung. Ein bevorzugter erfindungsgemäßer Gegenstand sind transgene Pflanze enthaltend eine erfindungsgemäße funktionelle oder nicht funktionelle (= Antisense-DNA oder enzymatische inaktives Enzym) Nukleinsäuresequenz oder eine funktionelle oder nicht funktionelle Expressionskassette.

Die Expressionskassette oder die erfindungsgemäßen Nukleinsäuresequenzen enthaltend eine Δ-4-Desaturasegensequenz kann darüber hinaus auch zur Transformation der oben beispielhaft genannten Organismen wie Bakterien, Cyanobakterien, Hefen, filamentösen Pilzen, Ciliaten und Algen mit dem Ziel einer Erhöhung des Gehaltes an Fettsäuren, Ölen oder Lipiden Δ-4-Doppelbindungen eingesetzt werden.

Erhöhung des Gehaltes von Fettsäuren, Ölen oder Lipiden mit Δ-4-Doppelbindungen bedeutet im Rahmen der vorliegenden Erfindung beispielsweise die künstlich erworbene Fähigkeit einer erhöhten Biosyntheseleistung durch funktionelle Überexpression des Δ-4-Desaturase-Gens in den erfindungsgemäßen Organismen vorteilhaft in den erfindungsgemäßen transgenen Pflanzen gegenüber den nicht gentechnisch modifizierten Ausgangspflanzen zumindest für die Dauer mindestens einer Pflanzengeneration.

Der Biosyntheseort von Fettsäuren, Ölen-oder Lipiden beispielsweise ist im allgemeinen der Samen oder Zellschichten des Samens, so dass eine samenspezifische Expression des Δ-4-Desaturase-Gens sinnvoll ist. Es ist jedoch naheliegend, dass die Biosynthese von Fettsäuren, Ölen oder Lipiden nicht auf das Samengewebe beschränkt sein muss, sondern auch in allen übrigen Teilen der Pflanze - beispielsweise in Epidermiszellen oder in den Knollen - gewebespezifisch erfolgen kann.

Darüber hinaus ist eine konstitutive Expression des exogenen Δ-4-Desaturase-Gens von Vorteil. Andererseits kann aber auch eine induzierbare Expression wünschenswert erscheinen.

Die Wirksamkeit der Expression des Δ-4-Desaturase-Gens kann beispielsweise *in vitro* durch Sprossmeristemvermehrung ermittelt werden. Zudem kann eine in Art und Höhe veränderte Expression des Δ-4-Desaturase-Gens und deren Auswirkung auf die Fettsäure-, Öl- oder Lipidbiosyntheseleistung an Testpflanzen in Gewächshausversuchen getestet werden.

Gegenstand der Erfindung sind außerdem transgene Pflanzen, transformiert mit einer Expressionskassette enthaltend eine Δ-4-Desaturase-Gensequenz oder mit dieser hybridisierende DNA-Sequenzen, sowie transgene Zellen, Gewebe, Teile und Vermehrungsgut solcher Pflanzen. Besonders bevorzugt sind dabei transgene Kulturpflanzen, wie z.B. Gerste, Weizen, Roggen, Hafer, Mais, Soja, Reis, Baumwolle, Zuckerrübe, Raps und Canola, Sonnenblume, Flachs, Hanf, Kartoffel, Tabak, Tomate, Raps, Tapioka, Maniok, Pfeilwurz, Alfalfa, Salat und die verschiedenen Baum-, Nuss und Weinspezies.

Pflanzen im Sinne der Erfindung sind mono- und dikotyle Pflanzen, Moose oder Algen.

Eine weitere erfindungsgemäße Ausgestaltung sind wie oben beschriebenen transgene Pflanzen, die eine funktionelle oder nicht funktionelle erfindungsgemäße Nukleinsäuresequenz oder eine funktionelle oder nicht funktionelle erfindungsgemäße Expressionskassette enthalten. Unter nicht funktionell ist zu verstehen, dass kein enzymatisch aktives Protein mehr synthetisiert wird. Außerdem ist unter nicht funktionellen Nukleinsäuren oder Nukleinsäurekonstrukten auch eine sogenannte Antisense-DNA zu verstehen, die zu transgenen Pflanzen führt, die eine Reduktion der enzymatischen Aktivität oder keine enzymatischen Aktivität aufweisen. Mit Hilfe der Antisense-Technik, speziell wenn die erfindungsgemäße Nukleinsäuresequenz mit anderen Fettsäuresynthesegene in der Antisense-DNA kombiniert wird, ist es möglich Triglyceride mit einem erhöhten Gehalt an gesättigten Fettsäuren bzw. gesättigte Fettsäuren zu synthetisieren. Unter transgenen Pflanzen sind einzelne Pflanzenzellen und deren Kulturen auf Festmedien oder in Flüssigkultur, Pflanzenteile und ganze Pflanzen zu verstehen.

Weitere Gegenstände der Erfindung sind:
- Verfahren zur Transformation einer Pflanze dadurch gekennzeichnet, dass man erfindungsgemäße Expressionskassetten enthaltend eine Δ-4-Desaturase-Gensequenz aus Primulaceen oder mit dieser hybridisierende DNA'-Sequenzen in eine Pflanzenzelle, in Kallusgewebe, eine ganze Pflanze oder Protoplasten von Pflanzen einbringt.
- Verwendung einer Δ-4-Desaturase-DNA-Gensequenz oder mit dieser hybridisierende DNA-Sequenzen zur Herstellung von Pflanzen mit erhöhtem Gehalt an Fettsäuren, Ölen oder Lipiden mit Δ-4-Doppelbindungen durch Expression dieser Δ-4-Desaturase DNA-Sequenz in Pflanzen.
- Proteine enthaltend die in SEQ ID NO: 2 dargestellten Aminosäuresequenzen.
- Verwendung der Proteine mit den Sequenzen SEQ ID NO: 2 zur Herstellung von ungesättigten Fettsäuren.

Ein weiterer erfindungsgemäßer Gegenstand ist ein Verfahren zur Herstellung von ungesättigten Fettsäuren, dadurch gekennzeichnet, dass man mindestens eine oben beschriebene erfindungsgemäße Nukleinsäuresequenz oder mindestens ein erfindungsgemäßes Nukleinsäurekonstrukt in einen bevorzugt Öl produzierenden Organismus bringt, diesen Organismus anzieht und dass in dem Organismus enthaltene öl isoliert und die im Öl enthaltenden Fettsäuren freisetzt. Diese ungesättigten Fettsäuren enthalten vorteilhaft Δ-4-Doppelbindungen. Die Fettsäuren können aus den Ölen bzw. Lipiden beispielsweise über eine basische Hydrolyse z.B. mit NaOH oder KOH freigesetzt werden.

Ein weiterer beschriebener Gegenstand ist ein Verfahren zur Herstellung von Triglyceriden mit einem erhöhten Gehalt an ungesättigten Fettsäuren, dadurch gekennzeichnet, dass man mindestens eine oben beschriebene erfindungsgemäße Nukleinsäuresequenz oder mindestens eine erfindungsgemäße Expressionskassette in einen Öl produzierenden Organismus bringt, diesen Organismus anzieht und dass in dem Organismus enthaltene Öl isoliert, gehört zu den Erfindungsgegenständen.

Ein weiterer beschriebener Gegenstand ist ein Verfahren zur Herstellung von Triglyceriden mit einem erhöhten Gehalt an ungesättigten Fettsäuren, indem man Triglyceride mit gesättigten oder ungesättigten oder gesättigten und ungesättigten Fettsäuren mit mindestens dem Protein, das durch die Sequenz SEQ ID NO: 1 kodiert wird, inkubiert. Vorteilhaft wird das Verfahren in Gegenwart von Verbindungen durchgeführt, die Reduktionsäquivalente aufnehmen oder abgeben können. Anschließend können die Fettsäuren aus den Triglyceriden freigesetzt werden.

Vorteilhaft werden im erfindungsgemäßen Verfahren transgene Pflanzen als Organismen verwendet. Diese Pflanzen enthalten die im erfindungsgemäßen Verfahren synthetisierten mehrfach ungesättigten Fettsäuren und können vorteilhaft direkt vermarktet werden ohne dass, die synthetisierten Öle, Lipide oder Fettsäuren isoliert werden müssen. Unter Pflanzen im erfindungsgemäßen Verfahren sind ganze Pflanzen sowie alle Pflanzenteile, Pflanzenorgane oder Pflanzenteile wie Blatt, Stiel, Samen, Wurzel, Knollen, Antheren, Fasern, Wurzelhaare, Stängel, Embryos, Kalli, Kotelydonen, Petiolen, Erntematerial, pflanzliches Gewebe, reproduktives Gewebe, Zellkulturen, die sich von der transgenen Pflanze abgeleiten und/oder dazu verwendet werden können, die transgene Pflanze hervorzubringen. Der Samen umfasst dabei alle Samenteile wie die Samenhüllen, Epidermis- und Samenzellen, Endosperm oder Embyrogewebe. Die im erfindungsgemäßen Verfahren hergestellten Verbindungen können aber auch aus den Organismen vorteilhaft Pflanzen in Form ihrer Öle, Fett, Lipide und/oder freien Fettsäuren isoliert werden. Durch dieses Verfahren hergestellte mehrfach ungesättigten Fettsäuren lassen sich durch Ernten der Organismen entweder aus der Kultur, in der sie wachsen, oder vom Feld ernten. Dies kann über Pressen oder Extraktion der Pflanzenteile bevorzugt der Pflanzensamen erfolgen. Dabei können die Öle, Fette, Lipide und/oder freien Fettsäuren durch sogenanntes kalt schlagen oder kalt pressen ohne Zuführung von Wärme durch Pressen gewonnen werden. Damit sich die Pflanzenteile speziell die Samen leichter aufschließen lassen, werden sie vorher zerkleinert, gedämpft oder geröstet. Die so vorbehandelten Samen können anschließend gepresst werden oder mit Lösungsmittel wie warmen Hexan extrahiert werden. Anschließend wird das Lösungsmittel wieder entfernt. Im Falle von Mikroorganismen werden diese nach Ernte beispielsweise direkt ohne weitere Arbeitsschritte extrahiert oder aber nach Aufschluss über verschiedene dem Fachmann bekannte Methoden extrahiert. Auf diese Weise können mehr als 96 % der im Verfahren hergestellten Verbindungen isoliert werden. Anschließend werden die so erhaltenen Produkte weiter bearbeitet, das heißt raffiniert. Dabei werden zunächst beispielsweise die Pflanzenschleime und Trübstoffe entfernt. Die sogenannte Entschleimung kann enzymatisch oder beispielsweise chemisch/physikalisch durch Zugabe von Säure wie Phosphor säure erfolgen. Anschließend werden die freien Fettsäuren durch Behandlung mit einer Base beispielsweise Natronlauge entfernt. Das erhaltene Produkt wird zur Entfernung der im Produkt verbliebenen Lauge mit Wasser gründlich gewaschen und getrocknet. Um die noch im Produkt enthaltenen Farbstoffe zu entfernen werden die Produkte einer Bleichung mit beispielsweise Bleicherde oder Aktivkohle unterzogen. Zum Schluss wird das Produkt noch beispielsweise mit Wasserdampf noch desodoriert.

Die im Verfahren hergestellten PUFAs fallen in den Organismen vorteilhaft in Form ihrer Öle, Lipide oder Fettsäuren oder Fraktionen davon an.

Beschrieben ist die Verwendung des Öls, Lipids, der Fettsäuren und/oder der Fettsäurezusammensetzung in Futtermitteln, Nahrungsmitteln, Kosmetika oder Pharmazeutika.

Eine weitere erfindungsgemäße Ausführungsform ist die Verwendung der erfindungsgemäßen Δ-4-Desaturase zum bevorzugten Δ-4 Desaturieren von Fettsäuren in der sn-2 Position gegenüber der sn-1 Position.

Unter dem Begriff "Öl", "Lipid" oder "Fett" wird ein Fettsäuregemisch verstanden, das ungesättigte, gesättigte, vorzugsweise veresterte Fettsäure(n) enthält. Bevorzugt ist, dass das Öl, Lipid oder Fett einen hohen Anteil an mehrfach ungesättigten freien oder vorteilhaft veresterten Fettsäure(n), insbesondere Linolsäure, γ-Linolensäure, Dihomo-y-linolensäure, Arachidonsäure, α-Linolensäure, Stearidonsäure, Eicosatetraensäure, Eicosapentaensäure, Docosapentaensäure oder Docosahexaensäure hat. Vorzugsweise ist der Anteil an ungesättigten veresterten Fettsäuren ungefähr 30 %, mehr bevorzugt ist ein Anteil von 50 %, noch mehr bevorzugt ist ein Anteil von 60 %, 70 %, 80 % oder mehr. Zur Bestimmung kann z.B. der Anteil an Fettsäure nach Überführung der Fettsäuren in die Methylestern durch Umesterung gaschromatographisch bestimmt werden. Das Öl, Lipid oder Fett kann verschiedene andere gesättigte oder ungesättigte Fettsäuren, z.B. Calendulasäure, Palmitin-, Palmitolein-, Stearin-, Ölsäure etc., enthalten. Insbesondere kann je nach Ausgangsorganismus der Anteil der verschiedenen Fettsäuren in dem Öl oder Fett schwanken.

Bei den im Verfahren hergestellten mehrfach ungesättigte Fettsäuren, handelt es sich beispielsweise um Sphingolipide, Phosphoglyceride, Lipide, Glycolipide, Phospholipide, Monoacylglycerin, Diacylglycerin, Triacylglycerin oder sonstige Fettsäureester.

Alkalibehandlung beispielsweise wässrige KOH oder NaOH oder saure Hydrolyse vorteilhaft in Gegenwart eines Alkohols wie Methanol oder Ethanol oder über eine enzymatische Abspaltung freisetzen und isolieren über beispielsweise Phasentrennung und anschließender Ansäuerung über z.B. H₂SO₄. Die Freisetzung der Fettsäuren kann auch direkt ohne die vorhergehend beschriebene Aufarbeitung erfolgen.

Die oben genannten Verfahren ermöglichen vorteilhaft die Synthese von Fettsäuren oder Triglyceriden mit einem erhöhten Gehalt an Fettsäuren mit Δ-4-Doppelbindungen.

Die oben genannten Verfahren ermöglichen vorteilhaft die Synthese von Fettsäuren oder Triglyceriden mit einem erhöhten Gehalt an Fettsäuren mit Δ-4-Doppelbindungen, wobei als Substrat für die Reaktion der Δ4-Desaturase bevorzugt Docosapentaensäure verwendet wird. Damit ermöglicht das oben genannte Verfahren vorteilhaft besonders die Synthese von Fettsäuren, wie zum Beispiel Docosahexaensäure.

Mit Hilfe der sogenannten Antisense-Technologie können in einem Verfahren auch Fettsäuren oder Triglyceride mit einem erhöhten Gehalt an gesättigten Fettsäuren hergestellt werden.

Als Organismen für die genannten Verfahren seien beispielhaft Pflanzen wie Arabidopsis, Primulaceen, Borage, Gerste, Weizen, Roggen, Hafer, Mais, Soja, Reis, Baumwolle, Zuckerrübe, Raps und Canola, Sonnenblume, Flachs, Hanf, Kartoffel, Tabak, Tomate, Raps, Tapioka, Maniok, Pfeilwurz, Alfalfa, Erdnuss, Rizinus, Kokosnuss, Ölpalme, Färbersaflor (Carthamus tinctorius) oder Kakaobohne, Mikroorganismen wie Pilze Mortierella, Saprolegnia oder Pythium, Bakterien wie die Gattung Escherichia, Cyanobakterien, Hefen wie die Gattung Saccharomyces, Algen oder Protozoen wie Dinoflagellaten wie Crypthecodinium genannt. Bevorzugt werden Organismen, die natürlicherweise Öle in größeren Mengen synthetisieren können wie Mikroorganismen wie Pilze wie Mortierella alpina, Pythium insidiosum oder Pflanzen wie Soja, Raps, Kokosnuss, Ölpalme, Färbersaflor, Rizinus, Calendula, Erdnuss, Kakaobohne oder Sonnenblume oder Hefen wie Saccharomyces cerevisiae, besonders bevorzugt werden Soja, Raps, Sonnenblume, Carthamus oder Saccharomyces cerevisiae.

Die in den Verfahren verwendeten Organismen werden je nach Wirtsorganismus in dem Fachmann bekannter Weise angezogen bzw. gezüchtet. Mikroorganismen werden in der Regel in einem flüssigen Medium, das eine Kohlenstoffquelle meist in Form von Zuckern, eine Stickstoffquelle meist in Form von organischen Stickstoffquel-len wie Hefeextrakt oder Salzen wie Ammoniumsulfat, Spurenelemente wie Eisen-, Mangan-, Magnesiumsalze und gegebenenfalls Vitamine enthält, bei Temperaturen zwischen 0°C und 100°C, bevorzugt zwischen 10°C bis 60°C unter Sauerstoffbegasung angezogen. Dabei kann der pH der Nährflüssigkeit auf einen festen Wert gehalten werden, das heißt während der Anzucht reguliert werden oder nicht. Die Anzucht kann batch weise, semi batch weise oder kontinuierlich erfolgen. Nährstoffe können zu Beginn der Fermentation vorgelegt oder semikontinuierlich oder kontinuierlich nach gefüttert werden.

Pflanzen werden nach Transformation zunächst wie oben beschrieben regeneriert und anschließend wie üblich angezüchtet bzw. angebaut.

Aus den Organismen werden nach Anzucht die Lipide in üblicherweise gewonnen. Hierzu können die Organismen nach Ernte zunächst aufgeschlossen werden oder direkt verwendet werden. Die Lipide werden vorteilhaft mit geeigneten Lösungsmitteln wie apolare Lösungsmittel wie Hexan oder Ethanol, Isopropanol oder Gemischen wie Hexan/Isopropanol, Phenol/Chloroform/Isoamylalkohol bei Temperaturen zwischen 0°C bis 80°C, bevorzugt zwischen 20°C bis 50°C extrahiert. Die Biomasse wird in der Regel mit einem Überschuss an Lösungsmittel extrahiert beispielsweise einem Überschuss von Lösungsmittel zu Biomasse von 1:4. Das Lösungsmittel wird anschließend beispielsweise über eine Destillation entfernt. Die Extraktion kann auch mit superkritischem CO₂ erfolgen. Nach Extraktion kann die restliche Biomasse beispielsweise über Filtration entfernt werden.

Das so gewonnene Rohöl kann anschließend weiter aufgereinigt werden, beispielsweise in dem Trübungen über das Versetzen mit polaren Lösungsmittel wie Aceton oder Chloroform und anschließender Filtration oder Zentrifugation entfernt werden. Auch eine weitere Reinigung über Säulen ist möglich.

Zur Gewinnung der freien Fettsäuren aus den Triglyceriden werden diese in üblicherweise verseift.

Ein weiterer Gegenstand der Erfindung sind ungesättigte Fettsäuren sowie Trigylceride mit einem erhöhten Gehalt an ungesättigten Fettsäuren, die nach den oben genannten Verfahren hergestellt wurden, sowie deren Verwendung zur Herstellung von Nahrungsmitteln, Tierfutter, Kosmetika oder Pharmazeutika. Hierzu werden diese den Nahrungsmitteln, dem Tierfutter, den Kosmetika oder Pharmazeutika in üblichen Mengen zugesetzt.

Die Erfindung und das technische Gebiet werden durch die folgenden Beispiele näher erläutert:

### Beispiele

### Beispiel 1: Allgemeine Klonierungsverfahren:

Die Klonierungsverfahren wie z.B. Restriktionsspaltungen, Agarose-Gelelektrophorese, Reinigung von DNA-Fragmenten, Transfer von Nukleinsäuren auf Nitrozellulose und Nylon Membranen, Verknüpfen von DNA-Fragmenten, Transformation von Escherichia coli Zellen, Anzucht von Bakterien und die Sequenzanalyse rekombinanter DNA wurden wie bei Sambrook et al. (1989) (Cold Spring Harbor Laboratory Press: ISBN 0-87969-309-6) beschrieben durchgeführt.

### Beispiel 2: Sequenzanalyse rekombinanter DNA:

Die Sequenzierung rekombinanter DNA-Moleküle erfolgte mit einem Laserfluoreszenz-DNA-Sequenzierer der Firma ABI nach der Methode von Sanger (Sanger et al. (1977) Proc. Natl. Acad. Sci. USA74, 5463-5467). Fragmente resultierend aus einer Polymerase Kettenreaktion wurden zur Vermeidung von Polymerasefehlern in zu exprimierenden Konstrukten sequenziert und überprüft.

### Beispiel 3: Klonierung der Δ-4-Desaturase aus Euglena gracilis

Euglena gracilis Stamm 1224-5/25 wurde erhalten von der Sammlung für Algenkulturen Göttingen (SAG). Zur Isolierung wurde der Stamm in Medium II (Calvayrac R and Douce R, FEBS Letters 7:259-262, 1970) für 4 Tage bei 23 °C unter einem Licht-/ Dunkelintervall von 8 h / 16 h (35 mol s-1 m-2 Lichtstärke) angezogen.

Gesamt-RNA von einer viertägigen Euglena Kultur wurde mit Hilfe des RNAeasy Kits der Firma Qiagen (Valencia, CA, US) isoliert. Aus der Gesamt-RNA wurde mit Hilfe von oligo-dT-Cellulose poly-A+ RNA (mRNA) isoliert (Sambrook et al., 1989). Die RNA wurde mit dem Reverse Transcription System Kit von Promega revers transcribiert und die synthetisierte cDNA in den lambda ZAP Vektor (lambda ZAP Gold, Stratagene) kloniert. Entsprechend Herstellerangaben wurde die cDNA zur Plasmid-DNA entpackt und Klone wurden zur Zufallssequenzierung ansequenziert. Eine Sequenz zeigte Ähnlichkeit zu Δ-4-Desaturasen. Die gefundene Sequenz wurde als Sonde für das Screening der Phagen-cDNA (2*10⁵ Plaques) verwendet. Nach zwei Screening-Runden konnte eine cDNA mit Vollänge-Sequenz identifiziert werden.

### Beispiel 4: Klonierung von Expressionsplasmiden zur heterologen Expression in Hefen

Die klonierte cDNA enthält zwei putative Startkodons, die in zwei offenen Leserahmen mit 9 Basen Unterschied resultieren. Nur der kürzere Leserahmen (SEQ ID NO: 1 zeigte später Aktivität. Folgendes Primerpaar wurde verwendet, um diesen Leserahmen in den Vektor pYES2 (Invitrogen) zu klonieren:
Forward: 5'-GGTACCATGTTGGTGCTGTTTGGCAA
Reverse: 5'-CTCGAGTTATGACTTTTTGTCCCCG

Zusammensetzung des PCR-Ansatzes (50 µL):
5,00 µl Template cDNA
5,00 µL 10x Puffer (Advantage-Polymerase)+ 25mM MgCl2
5,00 µL 2mM dNTP
1,25 µl je Primer (10 pmol/µL)
0,50 µl Advantage-Polymerase

Die Advantage-Polymerase von Clontech wurden eingesetzt.

Reaktionsbedingungen der PCR:
Anlagerungstemperatur:1 1 min 55°C
Denaturierungstemperatur: 1 min 94°C
Elongationstemperatur: 2 min 72°C
Anzahl der Zyklen: 35

Das PCR Produkt wurde für 2 h bei 37 °C mit den Restriktionsenzymen Kpnl und Xhol inkubiert. Der Hefe-Expressionsvektor pYES2 wurde in gleicherweise inkubiert. Anschließend wurde das 1638 bp große PCR Produkt sowie der Vektor durch Agarose-Gelelektrophorese aufgetrennt und die entsprechenden DNA-Fragmente ausgeschnitten. Die Aufreinigung der DNA erfolge mittels Qiagen Gel purification Kit gemäß Herstellerangaben. Anschließend wurden Vektor und Δ-4-Desaturase cDNA ligiert. Dazu wurde das Rapid Ligation Kit von Roche verwendet. Das entstandene Plasmid pYES2-EGD4-2 wurde durch Sequenzierung verifiziert und in den Saccharomyces Stamm SC334 durch Elektroporation (1500 V) transformiert. Anschließend wurden die Hefen auf Minimalmedium ohne Uracil ausplattiert. Zellen, die auf Minimalmedium ohne Uracil wachstumsfähig waren, enthalten damit das Plasmid pYES2-EGD4-2.

### Beispiel 5: Klonierung von Expressionsplasmiden zur Samen-spezifischen - Expression in Pflanzen

Für die Transformation von Pflanzen wurde ein weiterer Transformationsvektor auf Basis von pSUN-USP erzeugt. Dazu wurde mit folgendem Primerpaar Notl-Schnittstellen am 5' und 3'-Ende des kodierenden Sequenz eingefügt:.
Forward: 5'-GCGGCCGCATGTTGGTGCTGTTTGGCAA
Reverse: 5'-GCGGCCGCATGACTTTTTGTCCCCG

Zusammensetzung des PCR-Ansatzes (50 µL):
5,00 µL Template cDNA
5,00 µL 10x Puffer (Advantage-Polymerase)+ 25mM MgCl2
5,00 µL 2mM dNTP
1,25 µL je Primer (10 pmol/µL)
0,50 µL Advantage-Polymerase

Die Advantage-Polymerase von Clontech wurden eingesetzt.

Reaktionsbedingungen der PCR:
Anlagerungstemperatur: 1 min 55°C
Denaturierungstemperatur: 1 min 94°C
Elongationstemperatur: 2 min 72°C
Anzahl der Zyklen: 35

Das PCR Produkt wurde für 16 h bei 37°C mit den Restriktionsenzym Notl inkubiert. Der Pflanzen-Expressionsvektor pSUN300-USP wurde in gleicherweise inkubiert. Anschließend wurde das 1642 bp grosse PCR Produkt sowie der 7624 bp große Vektor durch Agarose-Gelelektrophorese aufgetrennt und die entsprechenden DNA-Fragmente ausgeschnitten. Die Aufreinigung der DNA erfolge mittels Qiagen Gel purification Kit gemäß Herstellerangaben. Anschließend wurden Vektor und Δ-4-Desaturase cDNA ligiert. Dazu wurde das Rapid Ligation Kit von Roche verwendet. Das entstandene Plasmid pSUN-EGD4-2 wurde durch Sequenzierung verifiziert.

pSUN300 ist ein Derivat des Plasmides pPZP (Hajdukiewicz,P, Svab, Z, Maliga, P., (1994) The small versatile pPZP family of Agrobacterium binary vectors forplant transformation. Plant Mol Biol 25:989-994). pSUN-USP entstand aus pSUN300, indem in pSUN300 ein USP-Promotor als EcoRI- Fragment inseriert wurde. Das Poly-adenylierungssignal ist das des Octopinsynthase-Gens aus dem A. tumefaciens Ti-Plasmid (ocs-Terminator, Genbank Accession V00088) (De Greve,H., Dhaese,P.; Seurinck,J., Lemmers,M., Van Montagu,M. and Schell,J. Nucleotide sequence and transcript map of the Agrobacterium tumefaciens Ti plasmid-encoded octopine synthase gene J. Mol. Appl. Genet. 1 (6), 499-511 (1982) Der USP-Promotor entspricht den Nukleotiden 1-684 (Genbank Accession X56240), wobei ein Teil der nichtcodierenden Region des USP-Gens im Promotor enthalten ist. Das 684 Basenpaar große Promotorfragment wurde mittels käuflichen T7-Standardprimer (Stratagene) und mit Hilfe eines synthetisierten Primers über eine PCR-Reaktion nach Standardmethoden amplifiziert. (Primersequenz: 5'-GTCGACCCGCGGACTAGTGGGCCCTCTAGACCCGGGGGATCC GGATCTGCTGGCTATGAA-3'). Das PCR-Fragment wurde mit EcoRI/Sall nachgeschnitten und in den Vektor pSUN300 mit OCS Terminator eingesetzt. Es entstand das Plasmid mit der Bezeichnung pSUN-USP. Das Konstrukt wurde zur Transformation von Arabidopsis thaliana, Raps, Tabak und Leinsamen verwendet.

### Beispiel 6: Erzeugung von transgenen Pflanzen

a) Erzeugung transgener Rapspflanzen (verändert nach Moloney et al., 1992, Plant Cell Reports, 8:238-242)
   Zur Erzeugung transgener Rapspflanzen wurden binäre Vektoren in Agrobacterium tumefaciens C58C1:pGV2260 oder Escherichia coli genutzt (Deblaere et al, 1984, Nucl. Acids. Res. 13,4777-4788). Zur Transformation von Rapspflanzen (Var. Drakkar, NPZ Norddeutsche Pflanzenzucht, Hohenlieth, Deutschland), wurde eine 1:50 Verdünnung einer Übernachtkultur einer positiv transformierten Agrobakterienkolonie in Murashige-Skoog Medium (Murashige und Skoog 1962 Physiol. Plant. 15, 473) mit 3 % Saccharose (3MS-Medium) benutzt. Petiolen oder Hypokotyledonen frisch gekeimter steriler Rapspflanzen (zu je ca. 1 cm²) wurden in einer Petrischale mit einer 1:50 Agrobakterienverdünnung für 5-10 Minuten inkubiert. Es folgt eine 3-tägige Colnkubation in Dunkelheit bei 25°C auf 3MS-Medium mit 0,8 % Bacto-Agar. Die Kultivierung wurde nach 3 Tagen mit 16 Stunden Licht /8 Stunden Dunkelheit weitergeführt und in wöchentlichem Rhythmus auf MS-Medium mit 500 mg/l Claforan (Cefotaxime-Natrium), 50 mg/l Kanamycin, 20 mikroM Benzylaminopurin (BAP) und 1,6 g/l Glukose weitergeführt. Wachsende Sprosse wurden auf MS-Medium mit 2 % Saccharose, 250 mg/l Claforan und 0,8 % Bacto-Agar überführt. Bildeten sich nach drei Wochen keine Wurzeln, so wurde als Wachstumshormon 2-Indolbuttersäure zum Bewurzeln zum Medium gegeben.
   Regenerierte Sprosse werden auf 2MS-Medium mit Kanamycin und Claforan erhalten, nach Bewurzelung in Erde überführt und nach Kultivierung für zwei Wochen in einer Klimakammer oder im Gewächshaus angezogen, zur Blüte gebracht, reife Samen geerntet und auf Δ-4--Desaturase -Expression mittels Lipidanalysen untersucht Linien mit erhöhten Gehalten an oder Doppelbindungen an der Δ-4-position werden identifiziert. Es lässt sich in den stabil transformierten transgenen Linien, die das Transgen funktionell exprimieren, ein erhöhter Gehalt von Doppelbindungen an der Δ-4-position im Vergleich zu untransformierten Kontrollpflanzen feststellen.
b) Die Herstellung von transgenen Leinpflanzen können zum Beispiel nach der Methode von Bell et al.,1999, In Vitro Cell. Dev. Biol.-Plant. 35(6):456-465 mittels particle bombartment erzeugt werden. Agrobakterien-vermittelte Transformationen können zum Beispiel nach Mlynarova et al. (1994), Plant Cell Report 13: 282-285 hergestellt werden.

### Beispiel 7: Lipidextraktion aus Hefen:

Hefen, die wie unter Beispiel 4 mit dem Plasmid pYES-EGD4-2 transformiert wurden, wurden folgendermaßen analysiert:
Die Hefezellen wurden für zwei Tage in 1 ml Minimalmedium mit 0,2 % Raffinose angezogen und dann in 5 ml desselben Mediums überführt. Diese Kultur wurde bis zu einer OD600 von 0,05 für 6 h bei 30 °C angezogen. Es wurden dann 100 µM der Fettsäure-Substrate (67 µM für 16:1 Δ7) zugegeben und die Expression der Δ-4-Desaturase durch Zugabe von 2 % Galaktose induziert. Die Zellen wurden dann 4 Tage bei 15 °C inkubiert, geerntet, gewaschen mit 100 mM NaHCO3 und für die Fettsäureanalyse mittels GC eingesetzt.

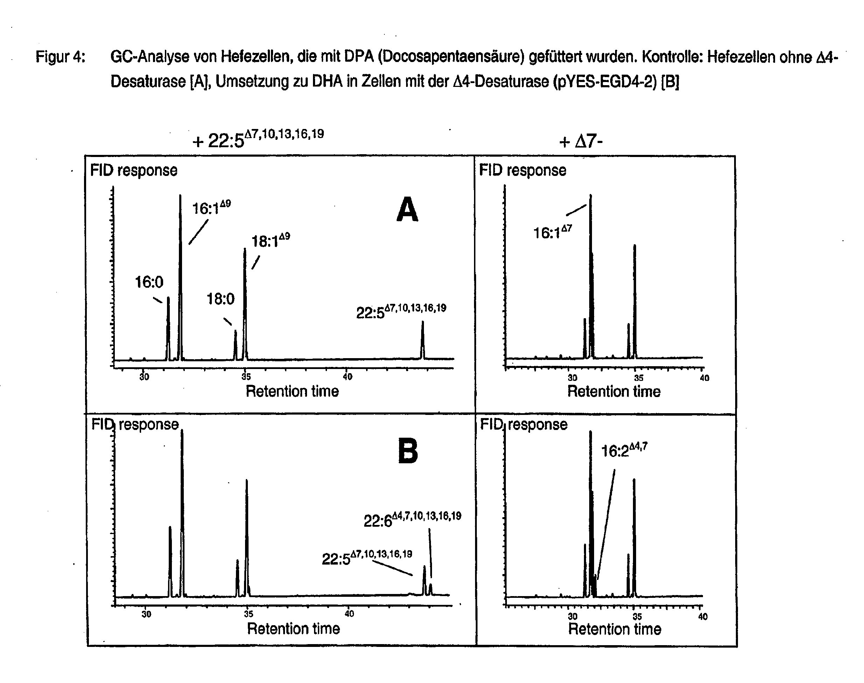

Fig. 4 zeigt das Ergebnis der Fettsäureanalyse. Dabei konnte gezeigt werden, dass im Vergleich zum Kontroll-Hefestamm, der nicht über das Δ-4-Desaturasegen verfügt, in dem Hefestamm mit dem pYES-EGD4-2 Konstrukt die gefütterte Fettsäure DPA (Docosapentaensäure) zu DHA (Docosahexaensäure) desaturiert wurde.

Die Substrat Spezifitäten wurden durch Fütterung der transformierten Hefestämme mit den unterschiedlichen Fettsäuren ermittelt (Tab. 1). Bestimmt wurde dann die Umsetzung der gefütterten Fettsäuren zu ihren Δ4 desaturierten Produkten.

Die Methodik ist beschrieben zum Beispiel in Napier and Michaelson, 2001,Lipids. 36(8):761-766; Sayanova et al., 2001, Journal of Experimental Botany. 52(360):1581-1585, Sperling et al., 2001, Arch. Biochem. Biophys. 388(2):293-298 und Michaelson et al., 1998, FEBS Letters. 439(3):215-218.

Die Analyse der Substratspezifität zeigte, dass die C7-C8 Doppelbindung notwendig ist für die Substraterkennung.

**Tabelle 1: Substratspezifität der Euglena gracilis Δ4-Desaturase**

| **Gefütterte Fettsäure** | **Davon umgesetzt (in %)** |
|---|---|
| 22:5 Δ7,10,13,16,19 | 29, 7 (±2,8) |
| 22:4 Δ7,10,13,16 | 28,7 (±2,5) |
| 20:3 Δ8,11,14 | Nicht detektierbar |
| 18:3 Δ6,9,12 | Nicht detektierbar |
| 18:2 Δ9,12 | Nicht detektierbar |
| 18:1 Δ9 | Nicht detektierbar |
| 18:0 | Nicht detektierbar |
| 16:3 Δ7,10,13 | 21,4 (±3,3) |
| 16:1 Δ7 | 7,4 (±0,8) |
| 16:1 Δ9 | Nicht detektierbar |
| 16:0 | Nicht detektierbar |

### Beispiel 8: Positionsanalyse der Δ4-desaturierten Fettsäuren

Neben der Substratspezifität wurde auch die Position der Δ-4-desaturierten Fettsäuren analysiert. Die Position von mehrfach ungesättigten Fettsäuren im Triacylglycerid ist von ernährungsphysiologischen Gesichtspunkten wichtig. Es wurde beschrieben, dass ungesättigte Fettsäuren besonders in der sn-2 Position von Triacylglyceriden schnell im Darm von Säugetieren aufgenommen werden. Um die Positionsspezifität der Δ-4-Desaturase aus Euglena gracilis zu untersuchen, wurde folgendermaßen vorgegangen:
Gemäß der Beschreibung in Beispiel 4 wurden 100 ml Hefekultur, die die Δ-4-Desaturase aus Hefe exprimieren, mit 16:1 Δ7 bzw. mit 22:4 Δ7,10,13,16 gefüttert und dann inkubiert. Die Gesamtlipide wurden aus den Hefen mittels Chforoform/Methanol/Wasser Extraktion isoliert (Bligh, E.G. and Dyer, W.J. Can J Biochem Physiol 37:911-917, 1959) und über Dünnschicht-Chromatographie aufgetrennt (Chloroform/Methanol/Essigsäure 65:25:8). Phosphatidylcholin wurden von den Dünnschichtplatten abgekratzt und mit 2 ml Chloroform/ Methanol (2/1) extrahiert. Das extrahiert Phosphatidylcholin wurde getrocknet und in 50 µL 100 % Trition-100 resuspendiert. Zu der Lösung wurde 1 ml 50 mM HEPES, 2 mM CaCl₂ und 10000 Einheiten Lipase (Rhizopus arrhizus delemar, Sigma) zugegeben. Nach 2 Stunden Inkubation bei 37 °C wurde die Lösung mit Essigsäure (100 %) acidifiziert und die Lipide und freie Fettsäuren mit Chloroform/ Methanol extrahiert. Die freien Fettsäuren sowie das entstandene Lysophosphatidylcholin wurden durch Dünnschichtchromatographie getrennt, von der Platte gekratzt und durch GC analysiert.

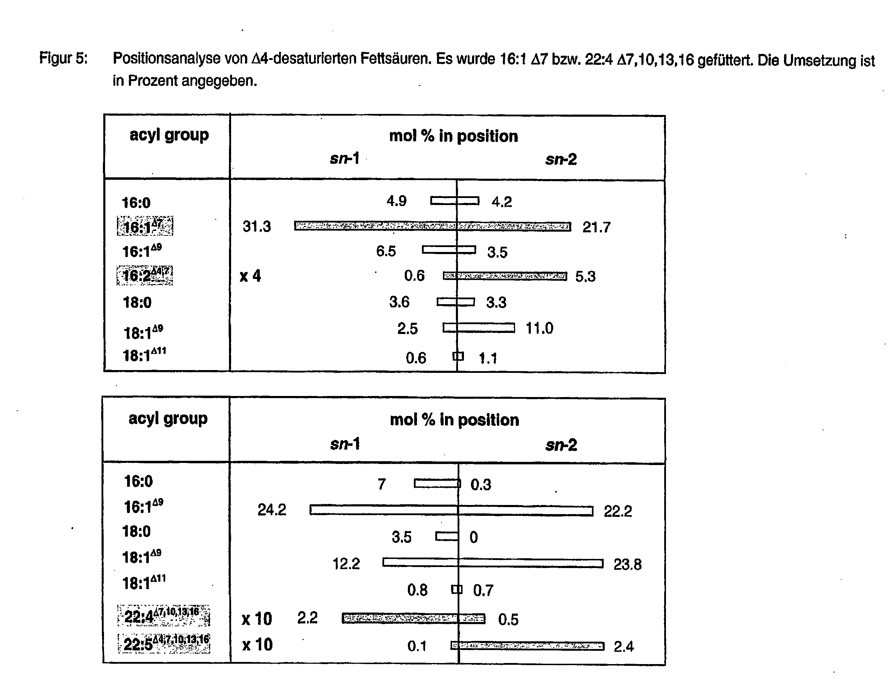

Die Ergebnisse sind in Fig. 5 dargestellt. Daraus geht hervor, das die sn-2 Position um den Faktor 20 gegenüber der sn-1 Position bevorzugt wird. Damit konnte gezeigt werden, dass die Desaturierung der Position C4-C5 zum größten Teil an Fettsäuren in der sn-2 Position erfolgt.

### Beispiel 9: Lipidextraktion aus Hefen und Samen:

Die Auswirkung der genetischen Modifikation in Pflanzen, Pilzen, Algen, Ciliaten oder auf die Produktion einer gewünschten Verbindung (wie einer Fettsäure) kann bestimmt werden, indem die modifizierten Mikroorganismen oder die modifizierte Pflanze unter geeigneten Bedingungen (wie den vorstehend beschriebenen) gezüchtet werden und das Medium und/oder die zellulären Komponenten auf die erhöhte Produktion des gewünschten Produktes (d.h. von Lipiden oder einer Fettsäure) untersucht wird. Diese Analysetechniken sind dem Fachmann bekannt und umfassen Spektroskopie, Dünnschichtchromatographie, Färbeverfahren verschiedener Art, enzymatische und mikrobiologische Verfahren sowie analytische Chromatographie, wie Hochleistungs-Flüssigkeitschromatographie (siehe beispielsweise Ullman, Encyclopedia of Industrial Chemistry, Bd. A2, S. 89-90 und S. 443-613, VCH: Weinheim (1985); Fallon, A., et al., (1987) "Applications of HPLC in Biochemistry" in: Laboratory Techniques in Biochemistry and Molecular Biology, Bd. 17; Rehm et al. (1993) Biotechnology, Bd. 3, Kapitel III: "Product recovery and purification", S. 469-714, VCH: Weinheim; Belter, P.A., et al. (1988) Bioseparations: downstream processing for Biotechnology, John Wiley and Sons; Kennedy, J.F., und Cabral, J.M.S. (1992) Recovery processes for biological Materials, John Wiley and Sons; Shaeiwitz, J.A., und Henry, J.D. (1988) Biochemical Separations, in: Ullmann's Encyclopedia of Industrial Chemistry, Bd. B3; Kapitel 11, S. 1-27, VCH: Weinheim; und Dechow, F.J. (1989) Separation and purification techniques in biotechnology, Noyes Publications).

Neben den oben erwähnten Verfahren werden Pflanzenlipide aus Pflanzenmaterial wie von Cahoon et al. (1999) Proc. Natl. Acad. Sci. USA 96 (22):12935-12940, und Browse et al. (1986) Analytic Biochemistry 152:141-145, beschrieben extrahiert. Die qualitative und quantitative Lipid- oder Fettsäureanalyse ist beschrieben bei Christie, William W., Advances in Lipid Methodology, Ayr/Scotland: Oily Press (Oily Press Lipid Library; 2); Christie, William W., Gas Chromatography and Lipids. A Practical Guide - Ayr, Scotland: Oily Press, 1989, Repr. 1992, IX, 307 S. (Oily Press Lipid Library; 1); "Progress in Lipid Research, Oxford: Pergamon Press, 1 (1952) -16 (1977) u.d.T.: Progress in the Chemistry of Fats and Other Lipids CODEN.

Zusätzlich zur Messung des Endproduktes der Fermentation ist es auch möglich, andere Komponenten der Stoffwechselwege zu analysieren, die zur Produktion der gewünschten Verbindung verwendet werden, wie Zwischen- und Nebenprodukte, um die Gesamteffizienz der Produktion der Verbindung zu bestimmen.

Die Analyseverfahren umfassen Messungen der Nährstoffmengen im Medium (z.B. Zucker, Kohlenwasserstoffe, Stickstoffquellen, Phosphat und andere Ionen), Messungen der Biomassezusammensetzung und des Wachstums, Analyse der Produktion üblicher Metabolite von Biosynthesewegen und Messungen von Gasen, die während der Fermentation erzeugt werden. Standardverfahren für diese Messungen sind in Applied Microbial Physiology; A Practical Approach, P.M. Rhodes und P.F. Stanbury, Hrsgb., IRL Press, S. 103-129; 131-163 und 165-192 (ISBN: 0199635773) und darin angegebenen Literaturstellen beschrieben.

Ein Beispiel ist die Analyse von Fettsäuren (Abkürzungen: FAME, Fettsäuremethylester; GC-MS, Gas-Flüssigkeitschromatographie-Massenspektrometrie; TAG, Triacylglycerin; TLC, Dünnschichtchromatographie).

Der unzweideutige Nachweis für das Vorliegen von Fettsäureprodukten kann mittels Analyse rekombinanter Organismen nach Standard-Analyseverfahren erhalten werden: GC, GC-MS oder TLC, wie verschiedentlich beschrieben von Christie und den Literaturstellen darin (1997, in: Advances on Lipid Methodology, Vierte Autl.: Christie, Oily Press, Dundee, 119-169; 1998, Gaschromatographie-Massenspektrometrie-Verfahren, Lipide 33:343-353).

Das zu analysierende Material kann durch Ultraschallbehandlung, Mahlen in der Glasmühle, flüssigen Stickstoff und Mahlen oder über andere anwendbare Verfahren aufgebrochen werden. Das Material muss nach dem Aufbrechen zentrifugiert werden. Das Sediment wird in Aqua dest. resuspendiert, 10 min bei 100°C erhitzt, auf Eis abgekühlt und erneut zentrifugiert, gefolgt von Extraktion in 0,5 M Schwefelsäure in Methanol mit 2 % Dimethoxypropan für 1 Std. bei 90°C, was zu hydrolysierten Öl- und Lipidverbindungen führt, die transmethylierte Lipide ergeben. Diese Fottsäuremethylester werden in Petrolether extrahiert und schließlich einer GC-Analyse unter Verwendung einer Kapillarsäule (Chrompack, WCOT Fused Silica, CP-Wax-52 CB, 25 mikrom, 0,32 mm) bei einem Temperaturgradienten zwischen 170°C und 240°C für 20 min und 5 min bei 240°C unterworfen. Die Identität der erhaltenen Fettsäuremethylester muss unter Verwendung von Standards, die aus kommerziellen Quellen erhältlich sind (d.h. Sigma), definiert werden.

Pflanzenmaterial wird zunächst mechanisch durch Mörsern homogenisiert, um es einer Extraktion zugänglicher zu machen.

Dann wird 10 min auf 100°C erhitzt und nach dem Abkühlen auf Eis erneut sedimentiert. Das Zellsediment wird mit 1 M methanolischer Schwefelsäure und 2 % Dimethoxypropan 1 h bei 90°C hydrolysiert und die Lipide transmethyliert. Die resultierenden Fettsäuremethylester (FAME) werden in Petrolether extrahiert. Die extrahierten FAME werden durch Gasflüssigkeitschromatographie mit einer Kapillarsäule (Chrompack, WCOT Fused Silica, CP-Wax-52 CB, 25 m, 0,32 mm) und einem Temperaturgradienten von 170°C auf 240°C in 20 min und 5 min bei 240°C analysiert. Die Identität der Fettsäuremethylester wird durch Vergleich mit entsprechenden FAME-Standards (Sigma) bestätigt. Die Identität und die Position der Doppelbindung kann durch , geeignete chemische Derivatisierung der FAME-Gemische z.B. zu 4,4-Dimethoxy-oxazolin-Derivaten (Christie, 1998) mittels GC-MS weiter analysiert werden.

## Patentansprüche

1. Isolierte Nukleinsäuresequenzen, die für Polypeptide mit Delta-4-Desaturaseaktivität codieren, ausgewählt aus der Gruppe:
a) einer Nukleinsäuresequenz mit der in SEQ ID NO: 1 dargestellten - Sequenz,
b) Nukleinsäuresequenzen, die sich als Ergebnis des degenerierten - genetischen Codes von der in SEQ ID NO: 1 enthaltenden codierenden Sequenz ableiten lassen, oder
c) Derivate der in SEQ ID NO: 1 dargestellten Nukleinsäuresequenz, die für Polypeptide mit der in SEQ ID NO: 2 dargestellten Aminosäuresequenzen codieren und mindestens 70 % Identität auf Aminosäureebene mit SEQ ID NO: 2 aufweisen und die Umsetzung folgender Fettsäuren zu ihren Delta-4 desaturierten Produkten bewirken können:
22:5 Delta-7,10,13,16,19
22:4 Delta-7,10,13,16
16:3 Delta-7,10,13
16:1 Delta-7

2. Isolierte Nukleinsäuresequenz nach Anspruch 1, wobei die Sequenz von einer Pflanze stammt.

3. Isolierte Nukleinsäuresequenz nach Anspruch 1 oder 2, wobei die Sequenz aus der Klasse der Euglenophyceae stammt.

4. Aminosäuresequenz, die von einer isolierten Nukleinsäuresequenz nach einem der Ansprüche 1 bis 3 codiert wird.

5. Genkonstrukt, enthaltend eine isolierte Nukleinsäure nach einem der Ansprüche 1 bis 3, wobei die Nukleinsäure funktionsfähig mit einem oder mehreren Regulationssignalen verbunden ist.

6. Genkonstrukt nach Anspruch 5, **dadurch gekennzeichnet, dass** das Nukleinsäurekonstrukt zusätzliche Biosynthesegene des Fettsäure- oder Lipidstoffwechsels enthält ausgewählt aus der Gruppe Acyl-CoA-Dehydrogenase(n), Acyl-ACP[= acyl carrier protein]-Desaturase(n), Acyl-ACP-Thioesterase(n), Fettsäure-Acyl-Transferase(n), Acyl-CoA:Lysophospholipid-Acyltransferase(n), Fettsäure-Synthase(n), Fettsäure-Hydroxylase(n), Acetyl-Coenzym A-Carboxylase(n), Acyl-Coenzym A-Oxidase(n), Fettsäure-Desaturase(n), Fettsäure-Acetylenasen, Lipoxygenasen, Triacylglycerol-Lipasen, Allenoxid-Synthasen, Hydropperoxid-Lyasen oder Fettsäure-Elongase(n).

7. Genkonstrukt nach Anspruch 5 oder 6, **dadurch gekennzeichnet, dass** das Nukleinsäurekonstrukt zusätzliche Biosynthesegene des Fettsäure- oder - Lipidstoffwechsels enthält ausgewählt aus der Gruppe der Delta-4-Desaturase-, Delta-5-Desaturase-, Delta-6-Desaturase-, Delta-8-Desatuase-, Delta-9-Desaturase-, Delta-12-Desaturase-, Delta-5-Elongase-, Delta-6-Elongase- oder Delta-9-Elongase.

8. Vektor, enthaltend eine Nukleinsäure nach den Ansprüchen 1 bis 3 oder ein Genkonstrukt nach Anspruch 5.

9. Transgener nicht-humaner Organismus, enthaltend mindestens eine Nukleinsäure nach den Ansprüchen 1 bis 3, ein Genkonstrukt nach Anspruch 5 oder einen Vektor nach Anspruch 8.

10. Transgener nicht-humaner Organismus nach Anspruch 8, wobei der Organismus ein Mikroorganismus, ein nicht-humanes Tier oder eine Pflanze ist.

11. Transgener nicht-humaner Organismus nach Anspruch 9 oder 10, wobei der Organismus eine Pflanze ist.

12. Verfahren zur Herstellung von mehrfach ungesättigten Fettsäuren, wobei das Verfahren das Züchten eines transgenen Organismus umfasst, der eine - Nukleinsäure nach den Ansprüchen 1 bis 3, ein Genkonstrukt nach Anspruch 5 oder einen Vektor nach Anspruch 8 umfasst, kodierend eine Delta-4-Desaturase, die spezifisch omega-3-Fettsäuren desaturiert, und wobei durch die Aktivität der Delta-4-Desaturase mehrfach ungesättigte Fettsäuren in dem Organismus gebildet werden, die einen erhöhten Gehalt an omega-3-Fettsäuren aufweisen.

13. Verfahren nach Anspruch 12, wobei im Verfahren Docosahexaensäure hergestellten wird.

14. Verfahren nach Anspruch 12 oder 13, wobei die mehrfach ungesättigten Fettsäuremoleküle aus dem Organismus in Form eines Öls, Lipids oder einer freien Fettsäure isoliert werden.

15. Verfahren nach einem der Ansprüche 12 bis 14, wobei der Organismus ein Mikroorganismus, ein nicht-humanes Tier oder eine Pflanze ist.

16. Verfahren nach einem der Ansprüche 12 bis 15, wobei der Organismus eine transgene Pflanze ist.

17. Verwendung einer Delta-4-Desaturase gemäß Anspruch 3 zum bevorzugten Delta-4-Desaturieren von Fettsäuren in der sn-2 Position gegenüber der sn-1 Position.

## Claims

1. Isolated nucleic acids which code for a polypeptide with delta-4 desaturase activity, selected from the group of
a) a nucleic acid sequence with the sequence shown in SEQ ID NO: 1,
b) nucleic acid sequences which, as the result of the degenerate genetic code, can be derived from the coding sequence present in SEQ ID NO: 1 or
c) derivatives of the nucleic acid sequence shown in SEQ ID NO: 1 which code for polypeptides with the amino acid sequences shown in SEQ ID NO: 2 and which have at least 70% identity with SEQ ID NO: 2 at the amino acid level and which can bring about the conversion of the following fatty acids into their delta-4 desaturated products;
22:5 delta-7, 10, 13, 16, 19
22:4 delta-7, 10, 13, 16
16:3 delta-7, 10, 13
16:1 delta 7.

2. The isolated nucleic acid sequence according to claim 1, wherein the sequence is derived from a plant.

3. The isolated nucleic acid sequence according to claim 1 or 2, wherein the sequence is derived from the class of the Euglenophyceae.

4. An amino acid sequence which is coded by an isolated nucleic acid sequence according to any of claims 1 to 3.

5. A gene construct, comprising an isolated nucleic acid according to any of claims 1 to 3, wherein the nucleic acid is linked operably to one or more regulatory signals.

6. The gene construct according to claim 5, wherein the nucleic acid construct comprises additional biosynthesis genes of the fatty acid or lipid metabolism, selected from the group acyl-CoA dehydrogenase(s), acyl-ACP[= acyl carrier protein] desaturase(s), acyl-ACP thioesterase(s), fatty acid acyl transferase(s), acyl-CoA: lysaphospholipid acyl transferase(s), fatty acid synthase(s), fatty acid hydroxylase(s), acetyl coenzyme A carboxylase(s), acyl-coenzyme A oxidase(s), fatty acid desaturase(s), fatty acid acetylenases, lipoxygenases, triacylglycerol lipases, allene oxide synthases, hydroperoxide lyases or fatty acid elongase(s).

7. The gene construct according to claim 5 or 6, wherein the nucleic acid construct comprises additional biosynthesis genes of the fatty acid or lipid metabolism, selected from the group delta-4 desaturase, delta-5 desaturase, delta-6 desaturase, delta-8 desaturase, delta-9 desaturase, delta-12 desaturase, delta-5 elongase, delta-6 elongase or delta-9 elongase.

8. A vector, comprising a nucleic acid according to claims 1 to 3 or a gene construct according to claim 5.

9. A transgenic non-human organism, comprising at least one nucleic acid according to claims 1 to 3, a gene construct according to claim 5 or a vector according to claim 8.

10. The transgenic non-human organism according to claim 8, wherein the organism is a microorganism, a non-human animal or a plant.

11. The transgenic non-human organism according to claim 9 or 10, wherein the organism is a plant.

12. A process for the production of polyunsaturated fatty acids, wherein the process comprises the culturing of a transgenic organism which comprises a nucleic acid according to claims 1 to 3, a gene construct according to claim 5 or a vector according to claim 8, coding for a delta-4 desaturase which specifically desaturates omega-3 fatty acids, and wherein, as a result of the activity of the delta-4 desaturase, polyunsaturated fatty acids with an increased content of omega-3 fatty acids are formed in the organism.

13. The process according to claim 12, wherein docosahexaenoic acid is produced in the process.

14. The process according to claim 12 or 13, wherein the polyunsaturated fatty acid molecules are isolated from the organism in the form of an oil, a liquid or a free fatty acid.

15. The process according to any of claims 12 to 14, wherein the organism is a microorganism, a non-human animal or a plant.

16. The process according to any of claims 12 to 15, wherein the organism is a transgenic plant.

17. The use of a delta-4 desaturase according to claim 3 for the preferential delta-4 desaturation of fatty acids in the sn-2 position over the sn-1 position.

## Revendications

1. Séquences d'acides nucléiques isolées, qui codent pour des polypeptides ayant une activité de delta-4-désaturase, choisies dans le groupe consistant en
a) une séquence d'acide nucléique ayant la séquence présentée dans SEQ ID NO:1,
b) les séquences d'acides nucléiques qui peuvent être obtenues en conséquence du code génétique dégénéré de la séquence codante contenue dans SEQ ID NO:1, ou
c) les dérivés de la séquence d'acide nucléique présentée dans SEQ ID NO:1, qui codent pour des polypeptides ayant la séquence d'acides aminés présentée dans SEQ ID NO:2, et qui ont une identité d'au moins 70 %, au niveau des acides aminés, avec SEQ ID NO:2,
et qui peuvent provoquer la conversion des acides gras suivants en leurs produits delta-4-désaturés .
22:5 delta-7,10,13,16,19
22:4 delta-7,10,13,16
16:3 delta-7,10,13
16:1 delta-7.

2. Séquence d'acides nucléiques isolée selon la revendication 1, la séquence provenant d'une plante.

3. Séquence d'acides nucléiques isolée selon la revendication 1 ou 2, la séquence provenant de la classe des Euglenophyceae.

4. Séquence d'acides aminés qui est codée par une séquence d'acide nucléique isolée selon l'une des revendications 1 à 3.

5. Construction génique contenant un acide nucléique isolé selon l'une des revendications 1 à 3, l'acide nucléique étant fonctionnellement lié à un ou plusieurs signaux de régulation.

6. Construction génique selon la revendication 5, **caractérisée en ce que** la construction d'acide nucléique contient des gènes supplémentaires de la biosynthèse du métabolisme des acides gras ou des lipides, choisis dans le groupe consistant en la ou les acyl-CoA-déshydrogénase(s), la ou les acyl-ACP[= protéine porteuse d'acyle]-désaturase(s), la ou les acyl-ACP-thioestérase(s), la ou les acide gras-acyl-transférase(s), les acyl-CoA:lysophospholipide-acyltransférase(s), les acide gras-synthase(s), les acide gras-hydroxylase(s), les acétyl-coenzyme-A-carboxylase(s), les acyl-coenzyme A-oxydase(s), les acide gras-désaturase(s), les acide gras-acétylénases, les lipoxygénases, les triacylglycérol-lipases, les allène-oxyde-synthases, les hydroperoxyde-lyases ou la ou les acide gras-élongase(s).

7. Construction génique selon 5 ou 6, **caractérisée en ce que** la construction d'acide nucléique contient des gènes supplémentaires de la biosynthèse du métabolisme des acides gras ou des lipides, choisis dans le groupe consistant en la delta-4-désaturase, la delta-5-désaturase, la delta-6-désaturase, la delta-8-désaturase, la delta-9-désaturase, la delta-12-désaturase, la delta-5-élongase, la delta-6-élongase ou la delta-9-élongase.

8. Vecteur contenant un acide nucléique selon la revendication 1 à 3 ou une construction génique selon la revendication 5.

9. Organisme transgénique non humain, contenant au moins un acide nucléique selon les revendications 1 à 3, une construction génique selon la revendication 5 ou un vecteur selon la revendication 8.

10. Organisme transgénique non humain selon la revendication 8, l'organisme étant un microorganisme, un animal non humain ou une plante.

11. Organisme transgénique non humain selon la revendication 9 ou 10, l'organisme étant une plante.

12. Procédé de préparation d'acides gras polyinsaturés, le procédé comprenant la culture d'un organisme transgénique qui comprend un acide nucléique selon les revendications 1 à 3, une construction génique selon la revendication 5 ou un vecteur selon la revendication 8, codant pour une delta-4-désaturase qui désature spécifiquement les acides gras oméga-3, et dans lequel, sous l'effet de l'activité de la delta-4-désaturase, il y a formation dans l'organisme d'acides gras polyinsaturés qui présentent une teneur accrue en acides gras oméga-3.

13. Procédé selon la revendication 12, de l'acide docosahexaénoïque étant préparé dans le procédé.

14. Procédé selon la revendication 12 ou 13, dans lequel les molécules d'acides gras polyinsaturés sont isolées de l'organisme sous forme d'une huile, d'un lipide ou d'un acide gras libre.

15. Procédé selon l'une des revendications 12 à 14, dans lequel l'organisme est un microorganisme, un animal non humain ou une plante.

16. Procédé selon l'une des revendications 12 à 15, dans lequel l'organisme est une plante transgénique.

17. Utilisation d'une delta-4-désaturase selon la revendication 3, pour une delta-4-désaturation d'acides gras de préférence en position sn-2 plutôt qu'en position sn-1.


### SEQUENCE LISTING

<110> BASF Plant Science GmbH
<120> Delta-4-Desaturasen aus Euglena gracilis, exprimierende Pflanzen und PUFA enthaltende Öle
<130> 20030192
<160> 2
<170> PatentIn version 3.1
<210> 1
   <211> 1626
   <212> DNA
   <213> Euglena gracilis
<220>
   <221> CDS
   <222> (1)..(1623)
   <223> Delta-4-Desaturase
<400> 1
   atg ttg gtg ctg ttt ggc aat ttc tat gtc aag caa tac tcc caa aag      48
   Met Leu Val Leu Phe Gly Asn Phe Tyr Val Lys Gln Tyr Ser Gln Lys
   1               5                   10                  15
   aac ggc aag ccg gag aac gga gcc acc cct gag aac gga gcg aag ccg      96
   Asn Gly Lys Pro Glu Asn Gly Ala Thr Pro Glu Asn Gly Ala Lys Pro
               20                  25                  30
   caa cct tgc gag aac ggc acg gtg gaa aag cga gag aat gac acc gcc     144
   Gln Pro Cys Glu Asn Gly Thr Val Glu Lys Arg Glu Asn Asp Thr Ala
           35                  40                  45
   aac gtt cgg ccc acc cgt cca gct gga ccc ccg ccg gcc acg tac tac     192
   Asn Val Arg Pro Thr Arg Pro Ala Gly Pro Pro Pro Ala Thr Tyr Tyr
       50                  55                  60
   gac tcc ctg gca gtg tcg ggg cag ggc aag gag cgg ctg ttc acc acc     240
   Asp Ser Leu Ala Val Ser Gly Gln Gly Lys Glu Arg Leu Phe Thr Thr
   65                  70                  75                  80
   gat gag gtg agg cgg cac atc ctc ccc acc gat ggc tgg ctg acg tgc      288
   Asp Glu Val Arg Arg His Ile Leu Pro Thr Asp Gly Trp Leu Thr Cys
                   85                  90                  95
   cac gaa gga gtc tac gat gtc act gat ttc ctt gcc aag cac cct ggt      336
   His Glu Gly Val Tyr Asp Val Thr Asp Phe Leu Ala Lys His Pro Gly
               100                 105                 110
   ggc ggt gtc atc acg ctg ggc ctt gga agg gac tgc aca atc ctc atc      384
   Gly Gly Val Ile Thr Leu Gly Leu Gly Arg Asp Cys Thr Ile Leu Ile
           115                 120                 125
   gag tca tac cac cct gct ggg cgc ccg gac aag gtg atg gag aag tac      432
   Glu Ser Tyr His Pro Ala Gly Arg Pro Asp Lys Val Met Glu Lys Tyr
       130                 135                 140
   cgc att ggt acg ctg cag gac ccc aag acg ttc tat gct tgg gga gag      480
   Arg Ile Gly Thr Leu Gln Asp Pro Lys Thr Phe Tyr Ala Trp Gly Glu
   145                 150                 155                 160
   tcc gat ttc tac cct gag ttg aag cgc cgg gcc ctt gca agg ctg aag      528
   Ser Asp Phe Tyr Pro Glu Leu Lys Arg Arg Ala Leu Ala Arg Leu Lys
                   165                 170                 175
   gag gct ggt cag gcg cgg cgc ggc ggc ctt ggg gtg aag gcc ctc ctg      576
   Glu Ala Gly Gln Ala Arg Arg Gly Gly Leu Gly Val Lys Ala Leu Leu
               180                 185                 190
   gtg ctc acc ctc ttc ttc gtg tcg tgg tac atg tgg gtg gcc cac aag      624
   Val Leu Thr Leu Phe Phe Val Ser Trp Tyr Met Trp Val Ala His Lys
           195                 200                 205
   tcc ttc ctc tgg gcc gcc gtc tgg ggc ttc gcc ggc tcc cac gtc ggg      672
   Ser Phe Leu Trp Ala Ala Val Trp Gly Phe Ala Gly Ser His Val Gly
       210                 215                 220
   ctg agc atc cag cac gat ggc aac cac ggc gcg ttc agc cgc aac aca      720
   Leu Ser Ile Gln His Asp Gly Asn His Gly Ala Phe Ser Arg Asn Thr
   225                 230                 235                 240
   ctg gtg aac cgc ctg gcg ggg tgg ggc atg gac ttg atc ggc gcg tcg      768
   Leu Val Asn Arg Leu Ala Gly Trp Gly Met Asp Leu Ile Gly Ala Ser
                   245                 250                 255
   tcc acg gtg tgg gag tac cag cac gtc atc ggc cac cac cag tac acc      816
   Ser Thr Val Trp Glu Tyr Gln His Val Ile Gly His His Gln Tyr Thr
               260                 265                 270
   aac ctc gtg tcg gac acg cta ttc agt ctg cct gag aac gat ccg gac      864
   Asn Leu Val Ser Asp Thr Leu Phe Ser Leu Pro Glu Asn Asp Pro Asp
           275                 280                 285
   gtc ttc tcc agc tac ccg ctg atg cgc atg cac ccg gat acg gcg tgg      912
   Val Phe Ser Ser Tyr Pro Leu Met Arg Met His Pro Asp Thr Ala Trp
   290 295 300
   cag ccg cac cac cgc ttc cag cac ctg ttc gcg ttc cca ctg ttc gcc 960
   Gln Pro His His Arg Phe Gln His Leu Phe Ala Phe Pro Leu Phe Ala
   305 310 315 320
   ctg atg aca atc agc aag gtg ctg acc agc gat ttc gct gtc tgc ctc 1008
   Leu Met Thr Ile Ser Lys Val Leu Thr Ser Asp Phe Ala Val Cys Leu
   325 330 335
   agc atg aag aag ggg tcc atc gac tgc tcc tcc agg ctc gtc cca ctg 1056
   Ser Met Lys Lys Gly Ser Ile Asp Cys Ser Ser Arg Leu Val Pro Leu
   340 345 350
   gag ggg cag ctg ctg ttc tgg ggg gcc aag ctg gcg aac ttc ctg ttg 1104
   Glu Gly Gln Leu Leu Phe Trp Gly Ala Lys Leu Ala Asn Phe Leu Leu
   355 360 365
   cag att gtg ttg cca tgc tac ctc cac ggg aca gct atg ggc ctg gcc 1152
   Gln Ile Val Leu Pro Cys Tyr Leu His Gly Thr Ala Met Gly Leu Ala
   370 375 380
   ctc ttc tct gtt gct cac ctt gtg tcg ggg gag tac ctc gcg atc tgc 1200
   Leu Phe Ser Val Ala His Leu Val Ser Gly Glu Tyr Leu Ala Ile Cys
   385 390 395 400
   ttc atc atc aac cac atc agc gag tct tgt gag ttt atg aat aca agc 1248
   Phe Ile Ile Asn His Ile Ser Glu Ser Cys Glu Phe Met Asn Thr Ser
   405 410 415
   ttt caa acc gcc gcc cgg agg aca gag atg ctt cag gca gca cat cag 1296
   Phe Gln Thr Ala Ala Arg Arg Thr Glu Met Leu Gln Ala Ala His Gln
   420 425 430
   gca gcg gag gcc aag aag gtg aag ccc acc cct cca ccg aac gat tgg 1344
   Ala Ala Glu Ala Lys Lys Val Lys Pro Thr Pro Pro Pro Asn Asp Trp
   435 440 445
   gct gtg aca cag gtc caa tgc tgc gtg aat tgg aga tca ggt ggc gtg 1392
   Ala Val Thr Gln Val Gln Cys Cys Val Asn Trp Arg Ser Gly Gly Val
   450 455 460
   ttg gcc aat cac ctc tct gga ggc ttg aac cac cag atc gag cat cat 1440
   Leu Ala Asn His Leu Ser Gly Gly Leu Asn His Gln Ile Glu His His
   465 470 475 480
   ctg ttc ccc agc atc tcg cat gcc aac tac ccc acc atc gcc cct gtt 1488
   Leu Phe Pro Ser Ile Ser His Ala Asn Tyr Pro Thr Ile Ala Pro Val
   485 490 495
   gtg aag gag gtg tgc gag gag tac ggg ttg ccg tac aag aat tac gtc 1536
   Val Lys Glu Val Cys Glu Glu Tyr Gly Leu Pro Tyr Lys Asn Tyr Val
   500 505 510
   acg ttc tgg gat gca gtc tgt ggc atg gtt cag cac ctc cgg ttg atg      1584
   Thr Phe Trp Asp Ala Val Cys Gly Met Val Gln His Leu Arg Leu Met
           515                 520                     525
   ggt gct cca ccg gtg cca acg aac ggg gac aaa aag tca taa              1626
   Gly Ala Pro Pro Val Pro Thr Asn Gly Asp Lys Lys Ser
       530                 535                 540
<210> 2
   <211> 541
   <212> PRT
   <213> Euglena gracilis
<400> 2
   Met Leu Val Leu Phe Gly Asn Phe Tyr Val Lys Gln Tyr Ser Gln Lys
   1               5                   10                  15
   Asn Gly Lys Pro Glu Asn Gly Ala Thr Pro Glu Asn Gly Ala Lys Pro
               20                  25                  30
   Gln Pro Cys Glu Asn Gly Thr Val Glu Lys Arg Glu Asn Asp Thr Ala
           35                  40                  45
   Asn Val Arg Pro Thr Arg Pro Ala Gly Pro Pro Pro Ala Thr Tyr Tyr
       50                  55                  60
   Asp Ser Leu Ala Val Ser Gly Gln Gly Lys Glu Arg Leu Phe Thr Thr
   65                  70                  75                  80
   Asp Glu Val Arg Arg His Ile Leu Pro Thr Asp Gly Trp Leu Thr Cys
                   85                  90                  95
   His Glu Gly Val Tyr Asp Val Thr Asp Phe Leu Ala Lys His Pro Gly
               100                 105                 110
   Gly Gly Val Ile Thr Leu Gly Leu Gly Arg Asp Cys Thr Ile Leu Ile
           115                 120                 125
   Glu Ser Tyr His Pro Ala Gly Arg Pro Asp Lys Val Met Glu Lys Tyr
       130                 135                 140
   Arg Ile Gly Thr Leu Gln Asp Pro Lys Thr Phe Tyr Ala Trp Gly Glu
   145                 150                 155                 160
   Ser Asp Phe Tyr Pro Glu Leu Lys Arg Arg Ala Leu Ala Arg Leu Lys
                   165                 170                 175
   Glu Ala Gly Gln Ala Arg Arg Gly Gly Leu Gly Val Lys Ala Leu Leu
               180                 185                 190
   Val Leu Thr Leu Phe Phe Val Ser Trp Tyr Met Trp Val Ala His Lys
           195                 200                 205
   Ser Phe Leu Trp Ala Ala Val Trp Gly Phe Ala Gly Ser His Val Gly
       210                 215                 220
   Leu Ser Ile Gln His Asp Gly Asn His Gly Ala Phe Ser Arg Asn Thr
   225                 230                 235                 240
   Leu Val Asn Arg Leu Ala Gly Trp Gly Met Asp Leu Ile Gly Ala Ser
                   245                 250                 255
   Ser Thr Val Trp Glu Tyr Gln His Val Ile Gly His His Gln Tyr Thr
               260                 265                 270
   Asn Leu Val Ser Asp Thr Leu Phe Ser Leu Pro Glu Asn Asp Pro Asp
           275                 280                 285
   Val Phe Ser Ser Tyr Pro Leu Met Arg Met His Pro Asp Thr Ala Trp
       290                 295                 300
   Gln Pro His His Arg Phe Gln His Leu Phe Ala Phe Pro Leu Phe Ala
   305                 310                 315                 320
   Leu Met Thr Ile Ser Lys Val Leu Thr Ser Asp Phe Ala Val Cys Leu
                   325                 330                 335
   Ser Met Lys Lys Gly Ser Ile Asp Cys Ser Ser Arg Leu Val Pro Leu
               340                 345                 350
   Glu Gly Gln Leu Leu Phe Trp Gly Ala Lys Leu Ala Asn Phe Leu Leu
           355                 360                 365
   Gln Ile Val Leu Pro Cys Tyr Leu His Gly Thr Ala Met Gly Leu Ala
       370                 375                 380
   Leu Phe Ser Val Ala His Leu Val Ser Gly Glu Tyr Leu Ala Ile Cys
   385                 390                 395                 400
   Phe Ile Ile Asn His Ile Ser Glu Ser Cys Glu Phe Met Asn Thr Ser
                   405                 410                 415
   Phe Gln Thr Ala Ala Arg Arg Thr Glu Met Leu Gln Ala Ala His Gln
               420                 425                 430
   Ala Ala Glu Ala Lys Lys Val Lys Pro Thr Pro Pro Pro Asn Asp Trp
           435                 440                 445
   Ala Val Thr Gln Val Gln Cys Cys Val Asn Trp Arg Ser Gly Gly Val
       450                 455                 460
   Leu Ala Asn His Leu Ser Gly Gly Leu Asn His Gln Ile Glu His His
   465                 470                 475                 480
   Leu Phe Pro Ser Ile Ser His Ala Asn Tyr Pro Thr Ile Ala Pro Val
                   485                 490                 495
   Val Lys Glu Val Cys Glu Glu Tyr Gly Leu Pro Tyr Lys Asn Tyr Val
               500                 505                 510
   Thr Phe Trp Asp Ala Val Cys Gly Met Val Gln His Leu Arg Leu Met
           515                 520                 525
   Gly Ala Pro Pro Val Pro Thr Asn Gly Asp Lys Lys Ser
       530                 535                 540